# EUROPEAN PATENT APPLICATION

(11) **EP 1 557 429 A1**
(43) Date of publication of application: **27.07.2005**
(21) Application number: 04001454.0
(22) Date of filing: 23.01.2004
(51) Int. Cl.: C07K 14/485, C07K 17/10

(54) **Site-directed coupling of proteins**

(71) Applicant: Vectron Therapeutics AG, 35037 Marburg (DE)
(72) Inventor: Konterman, Roland, Dr., 35085 Ebsdorfergund (DE)
(74) Representative: Zwicker, Jörk

(57) **Abstract**

The present invention relates to a method of modifying a polypeptide, which yields a modified polypeptide, suitable for site-specific coupling, for example, as a targeting ligand, as well as modified human EGF and fragments thereof, suitable for site-specific coupling.

SEQUENCE LISTING

<110>  vectron therapeutics

<120>  Site-directed coupling of proteins

<130>  FB13265

<160>  37

<170>  PatentIn version 3.2

<210>  1
<211>  53
<212>  PRT
<213>  Artificial

<220>
<223>  Selected EGF-binding peptide

<400>  1

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Glu Tyr Ala Cys Asn
            20                  25                  30

Cys Val Val Gly Tyr Ile Gly Glu Arg Cys Gln Tyr Arg Asp Leu Arg
        35                  40                  45

Trp Trp Glu Leu Arg
    50

<210>  2
<211>  53
<212>  PRT
<213>  Artificial

<220>
<223>  Selected EGF-binding peptide

<400>  2

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Gln Tyr Ala Cys Asn
            20                  25                  30

Cys Val Val Gly Tyr Ile Gly Glu Arg Cys Gln Tyr Arg Asp Leu Ser
        35                  40                  45

Trp Trp Glu Leu Arg
    50

<210>  3
<211>  53
<212>  PRT
<213>  Artificial

<220>
<223>  Selected EGF-binding peptide

<400>  3

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Ala Tyr Ala Cys Asn
            20                  25                  30

Cys Val Val Gly Tyr Ile Gly Glu Arg Cys Gln Tyr Arg Asp Leu Ala
        35                  40                  45

Trp Trp Glu Leu Arg
    50

<210>  4
<211>  53
<212>  PRT
<213>  Artificial

<220>
<223>  Selected EGF-binding peptide

<400>  4

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Ala Tyr Ala Cys Asn
            20                  25                  30

Cys Val Val Gly Tyr Ile Gly Glu Arg Cys Gln Tyr Arg Asp Leu Leu
        35                  40                  45

Trp Trp Glu Leu Arg
    50

<210>  5
<211>  53
<212>  PRT
<213>  Artificial

<220>
<223>  Selected EGF-binding peptide

<400>  5

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Val Tyr Ala Cys Asn
            20                  25                  30

Cys Val Val Gly Tyr Ile Gly Glu Arg Cys Gln Tyr Arg Asp Leu Leu
        35                  40                  45

Trp Trp Glu Leu Arg
    50

<210>  6
<211>  53
<212>  PRT
<213>  Artificial

<220>
<223>  Selected EGF-binding peptide

<400>  6

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Leu Tyr Ala Cys Asn
            20                  25                  30

Cys Val Val Gly Tyr Ile Gly Glu Arg Cys Gln Tyr Arg Asp Leu Leu
        35                  40                  45

Trp Trp Glu Leu Arg
    50

<210>  7
<211>  53
<212>  PRT
<213>  Artificial

<220>
<223>  Selected EGF-binding peptide

<400>  7

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Val Tyr Ala Cys Asn
            20                  25                  30

Cys Val Val Gly Tyr Ile Gly Glu Arg Cys Gln Tyr Arg Asp Leu Gly
        35                  40                  45

Trp Trp Glu Leu Arg
    50

<210>  8
<211>  53
<212>  PRT
<213>  Artificial

<220>
<223>  Selected EGF-binding peptide

<400>  8

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Phe Tyr Ala Cys Asn
            20                  25                  30

Cys Val Val Gly Tyr Ile Gly Glu Arg Cys Gln Tyr Arg Asp Leu Phe
        35                  40                  45

Trp Trp Glu Leu Arg
    50

<210>  9
<211>  53
<212>  PRT
<213>  Artificial

<220>
<223>  Selected EGF-binding peptide

<400>  9

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Ala Tyr Ala Cys Asn
            20                  25                  30

Cys Val Val Gly Tyr Ile Gly Glu Arg Cys Gln Tyr Asp Asp Leu Pro
        35                  40                  45

Trp Trp Glu Leu His
    50

<210>  10
<211>  53
<212>  PRT
<213>  Artificial

<220>

<223>  Selected EGF-binding peptide

<400>  10

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Ser Tyr Ala Cys Asn
            20                  25                  30

Cys Val Val Gly Tyr Ile Gly Glu Arg Cys Gln Tyr Arg Asp Leu Ala
        35                  40                  45

Trp Trp Glu Leu Pro
    50

<210>  11
<211>  53
<212>  PRT
<213>  Artificial

<220>
<223>  Selected EGF-binding peptide

<400>  11

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Ser Tyr Ala Cys Asn
            20                  25                  30

Cys Val Val Gly Tyr Ile Gly Glu Arg Cys Gln Tyr Ser Asp Leu Ala
        35                  40                  45

Trp Trp Glu Leu Asp
    50

<210>  12
<211>  53
<212>  PRT
<213>  Artificial

<220>
<223>  Selected EGF-binding peptide

<400>  12

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Gln Tyr Ala Cys Asn
            20                  25                  30

Cys Val Val Gly Tyr Ile Gly Glu Arg Cys Gln Tyr Ser Asp Leu Ser
        35                  40                  45

Trp Trp Glu Leu Ser
    50

<210>  13
<211>  53
<212>  PRT
<213>  Artificial

<220>
<223>  Selected EGF-binding peptide

<400>  13

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Gln Tyr Ala Cys Asn
            20                  25                  30

Cys Val Val Gly Tyr Ile Gly Glu Arg Cys Gln Tyr Ser Asp Leu Asn
        35                  40                  45

Trp Trp Glu Leu Ala
    50

<210>  14
<211>  53
<212>  PRT
<213>  Artificial

<220>
<223>  Selected EGF-binding peptide

<400>  14

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Gln Tyr Ala Cys Asn
            20                  25                  30

Cys Val Val Gly Tyr Ile Gly Glu Arg Cys Gln Tyr Ala Asp Leu Ala
        35                  40                  45

Trp Trp Glu Leu Gly
    50

<210>  15
<211>  53

<212>  PRT
<213>  Artificial

<220>
<223>  Selected EGF-binding peptide

<400>  15

Asn Ser Asp Ser Glu Cys Pro Leu Ser His Asp Gly Tyr Cys Leu His
1               5                   10                  15

Asp Gly Val Cys Met Tyr Ile Glu Ala Leu Asp Gln Tyr Ala Cys Asn
            20                  25                  30

Cys Val Val Gly Tyr Ile Gly Glu Arg Cys Gln Tyr Asn Asp Leu Pro
        35                  40                  45

Trp Trp Glu Leu Ser
    50

<210>  16
<211>  159
<212>  DNA
<213>  Artificial

<220>
<223>  DNA encoding selected EGF-binding peptide

<400>  16
aatagtgact ctgaatgtcc cctgtcccac gatgggtact gcctccatga tggtgtgtgc      60

atgtatattg aagctttgga cgagtatgca tgcaactgtg ttgttggcta catcggggag     120

cgatgtcagt accgagacct gcggtggtgg gaactgcgc                            159

<210>  17
<211>  159
<212>  DNA
<213>  Artificial

<220>
<223>  DNA encoding selected EGF-binding peptide

<400>  17
aatagtgact ctgaatgtcc cctgtcccac gatgggtact gcctccatga tggtgtgtgc      60

atgtatattg aagctttgga ccagtatgca tgcaactgtg ttgttggcta catcggggag     120

cgatgtcagt accgagacct gtcgtggtgg gaactgcgc                            159

<210>  18
<211>  159
<212>  DNA
<213>  Artificial

<220>
<223>  DNA encoding selected EGF-binding peptide

<400>  18
aatagtgact ctgaatgtcc cctgtcccac gatgggtact gcctccatga tggtgtgtgc      60

atgtatattg aagctttgga cgcttatgca tgcaactgtg ttgttggcta catcggggag     120

cgatgtcagt accgagacct ggcttggtgg gaactgcgc                            159


<210>  19
<211>  159
<212>  DNA
<213>  Artificial

<220>
<223>  DNA encoding selected EGF-binding peptide

<400>  19
aatagtgact ctgaatgtcc cctgtcccac gatgggtact gcctccatga tggtgtgtgc      60

atgtatattg aagctttgga cgcttatgca tgcaactgtg ttgttggcta catcggggag     120

cgatgtcagt accgagacct gctttggtgg gaactgcgc                            159


<210>  20
<211>  159
<212>  DNA
<213>  Artificial

<220>
<223>  DNA encoding selected EGF-binding peptide

<400>  20
aatagtgact ctgaatgtcc cctgtcccac gatgggtact gcctccatga tggtgtgtgc      60

atgtatattg aagctttgga cgtgtatgca tgcaactgtg ttgttggcta catcggggag     120

cgatgtcagt accgagacct gctttggtgg gaactgcgc                            159


<210>  21
<211>  159
<212>  DNA
<213>  Artificial

<220>
<223>  DNA encoding selected EGF-binding peptide

<400>  21
aatagtgact ctgaatgtcc cctgtcccac gatgggtact gcctccatga tggtgtgtgc      60

atgtatattg aagctttgga cctgtatgca tgcaactgtg ttgttggcta catcggggag     120

cgatgtcagt accgagacct gctttggtgg gaactgcgc                            159


<210>  22
<211>  159
<212>  DNA
<213>  Artificial

<220>

<223>  DNA encoding selected EGF-binding peptide

<400>  22
aatagtgact ctgaatgtcc cctgtcccac gatgggtact gcctccatga tggtgtgtgc      60

atgtatattg aagctttgga cgtgtatgca tgcaactgtg ttgttggcta catcggggag     120

cgatgtcagt accgagacct ggtttggtgg gaactgcgc                            159


<210>  23
<211>  159
<212>  DNA
<213>  Artificial

<220>
<223>  DNA encoding selected EGF-binding peptide

<400>  23
aatagtgact ctgaatgtcc cctgtcccac gatgggtact gcctccatga tggtgtgtgc      60

atgtatattg aagctttgga cttgtatgca tgcaactgtg ttgttggcta catcggggag     120

cgatgtcagt accgagacct gttgtggtgg gaactgcgc                            159


<210>  24
<211>  159
<212>  DNA
<213>  Artificial

<220>
<223>  DNA encoding selected EGF-binding peptide

<400>  24
aatagtgact ctgaatgtcc cctgtcccac gatgggtact gcctccatga tggtgtgtgc      60

atgtatattg aagctttgga cgcttatgca tgcaactgtg ttgttggcta catcggggag     120

cgatgtcagt acgatgacct gccttggtgg gaactgcat                            159


<210>  25
<211>  159
<212>  DNA
<213>  Artificial

<220>
<223>  DNA encoding selected EGF-binding peptide

<400>  25
aatagtgact ctgaatgtcc cctgtcccac gatgggtact gcctccatga tggtgtgtgc      60

atgtatattg aagctttgga cagttatgca tgcaactgtg ttgttggcta catcggggag     120

cgatgtcagt accgtgacct ggcttggtgg gaactgcct                            159


<210>  26
<211>  159
<212>  DNA
<213>  Artificial

<220>
<223>  DNA encoding selected EGF-binding peptide

<400>  26
aatagtgact ctgaatgtcc cctgtcccac gatgggtact gcctccatga tggtgtgtgc      60

atgtatattg aagctttgga cagttatgca tgcaactgtg ttgttggcta catcggggag     120

cgatgtcagt acagtgacct ggcttggtgg gaactggat                            159


<210>  27
<211>  159
<212>  DNA
<213>  Artificial

<220>
<223>  DNA encoding selected EGF-binding peptide

<400>  27
aatagtgact ctgaatgtcc cctgtcccac gatgggtact gcctccatga tggtgtgtgc      60

atgtatattg aagctttgga ccagtatgca tgcaactgtg ttgttggcta catcggggag     120

cgatgtcagt acagtgacct gagttggtgg gaactgagt                            159


<210>  28
<211>  159
<212>  DNA
<213>  Artificial

<220>
<223>  DNA encoding selected EGF-binding peptide

<400>  28
aatagtgact ctgaatgtcc cctgtcccac gatgggtact gcctccatga tggtgtgtgc      60

atgtatattg aagctttgga ccagtatgca tgcaactgtg ttgttggcta catcggggag     120

cgatgtcagt acagtgacct gaattggtgg gaactggct                            159


<210>  29
<211>  159
<212>  DNA
<213>  Artificial

<220>
<223>  DNA encoding selected EGF-binding peptide

<400>  29
aatagtgact ctgaatgtcc cctgtcccac gatgggtact gcctccatga tggtgtgtgc      60

atgtatattg aagctttgga ccagtatgca tgcaactgtg ttgttggcta catcggggag     120

cgatgtcagt acgctgacct ggcttggtgg gaactgggt                            159


<210>  30
<211>  159
<212>  DNA
<213>  Artificial

<220>
<223>  DNA encoding selected EGF-binding peptide

<400>  30
aatagtgact ctgaatgtcc cctgtcccac gatgggtact gcctccatga tggtgtgtgc      60

atgtatattg aagctttgga ccagtatgca tgcaactgtg ttgttggcta catcggggag     120

cgatgtcagt acaatgacct gccttggtgg gaactgagt                            159


<210>  31
<211>  15
<212>  PRT
<213>  Artificial

<220>
<223>  EGF1 fragment

<400>  31

Leu Val Phe Tyr Trp Arg His Asp Glu Cys Gln Ser Gly Ala Pro
1               5                   10                  15


<210>  32
<211>  9
<212>  PRT
<213>  Artificial

<220>
<223>  EGF2 fragment

<400>  32

Arg His Thr Ser Asp Asn Gly Ala Pro
1               5


<210>  33
<211>  42
<212>  DNA
<213>  Artificial

<220>
<223>  EGF amplification primer

<400>  33
tatgcggccc agccggccat ggccaatagt gactctgaat gt                        42


<210>  34
<211>  24
<212>  DNA
<213>  Artificial

<220>
<223>  EGF amplification primer

<400>  34
gtccaaagct tcaatataca tgca                                            24

<210>  35
<211>  36
<212>  DNA
<213>  Artificial

<220>
<223>  EGF mutation primer


<220>
<221>  misc_feature
<222>  (17)..(17)
<223>  n is a, c, g, or t

<400>  35
attgaagctt tggacbnkta tgcatgcaac tgtggt                                36


<210>  36
<211>  48
<212>  DNA
<213>  Artificial

<220>
<223>  EGF mutation primer


<220>
<221>  misc_feature
<222>  (29)..(29)
<223>  n is a, c, g, or t

<400>  36
cagtgcggcg cgacgcagtt cccaccamnv caggtctcgg tactgaca                   48


<210>  37
<211>  69
<212>  DNA
<213>  Artificial

<220>
<223>  EGF mutation primer

<400>  37
cagtgcggcc gcabbcagtt cccaccaabb caggtcabbg tactgacaab bctccccgat      60

gtagccaac                                                              69

## Description

### Background of the Invention

### 1. Field of the Invention

The present invention relates to a method of modifying a polypeptide, which yields a modified polypeptide, suitable for site-specific coupling, for example, as a targeting ligand, as well as modified human EGF and fragments thereof, suitable for site-directed coupling.

### 2. Description of Related Art

Therapeutics or diagnostics, which exhibit a tissue or cell specificity have been studied intensively in the past since they promise to provide an opportunity for the selective and efficient delivery of compounds to target cells or tissues. By now hundreds of different cell surface receptors or structures have been identified, many of which are differentially expressed on cells of different lineage and differentially on diseased versus healthy tissues or cells. These extracellular structures often specifically bind to or are specifically bound by so called "ligands". The term ligands encompasses a wide variety of substances, which can potentially bind to such surface structures, and include small signaling molecules as NO₂, hormones, drugs, small polypeptide signaling molecules, in particular cytokines and growth factors, and antibodies and fragments thereof. Ligand-targeted approaches require the attachment of ligands to the compounds to be targeted. Examples of such attachment include the chemical coupling of ligands to drugs, drug carrier system or other useful therapeutic or diagnostic compounds.

One particular interesting example is the use of polypeptides for the active targeting in cancer therapy. The concept of actively targeting drugs to tumor cells has led to the development of highly selective and efficacious anticancer therapeutics with reduced side effects (Allen (2002) Nature Reviews 2:750-763). Tumor cell specificity is conferred by ligands conjugated to the drug or drug carrier system. A wide variety of ligands have been explored including natural ligands, antibodies, peptides, and carbohydrates (Forssen & Willis (1998) Adv. Drug Deliv. Rev. 29:249-271; 1998; Allen (2002) Nature Reviews 2:750-763). Recent studies have shown that binding and subsequent internalization of actively targeted therapeutics by the target cells is a prerequisite to induce specific and efficacious antitumor effects (Park et al., (2002) Clin. Cancer Res. 8:1172-1181). Natural ligands such as growth factors and cytokines recognize cell surface-displayed receptors leading to receptor activation and internalization. Thus, these molecules are ideally suited for the generation of targeted internalizing drug systems.

Ligands are routinely conjugated to drugs or carrier systems by chemical coupling. However, the presence of several reactive groups at the ligand surface can lead to partial or complete inactivation or aggregation of the peptides or polypeptides and the generation of heterogeneous products due to multiple cross-linking between ligands and/or carriers or to ligand inactivation due to coupling at or near the active site. Thus, it would be advantageous to direct coupling to a single position which does not interfere with binding and internalization.

One possibility is to genetically introduce a reactive group, such as a cysteine residue containing a reactive sulfhydryl group, at a defined position, e.g. the N- or C-terminus. This approach was already applied to conjugate single-chain Fv fragments containing an additional C-terminal cysteine residue to liposomal carrier systems (Nielsen et al., (2002) Biochim. Biophys. Acta 1591:109-118); Marty et al., (2002) Br. J. Cancer 87:106-112). However, as in the case of EGF, ligands may contain already several cysteine residues which complicates expression and purification of correctly folded ligands. Furthermore, the free sulfhydryl group leads to dimerization due to cross-linking of two ligands, which requires reduction of the purified ligand under mild conditions prior to coupling.

An alternative approach is the coupling via the N-terminal amino group. This approach is, however, only feasible if the ligand does not contain additional amino groups, i.e. ε amino groups of lysine residues. Such an approach was realized by the group of Wagner using mouse EGF, which is naturally devoid of lysine residues, for coupling to polyethylenimine/DNA complexes through the bifunctional SPDP cross-linking reagent (Blessing et al., (2001) Bioconjug. Chem. 12:529-537). In another study, mouse EGF was used to produce targeted liposomes by activating EGF with Traut's reagent and coupling to maleimide-PEG lipids (Kullberg et al., (2002) Bioconjug. Chem. 13:737-743; Kullberg et al., (2003) Pharm. Res. 20, 229-236). However, for therapeutic applications it would be advantageous to use human EGF in order to avoid the induction of a neutralizing human anti-mouse immune response. In addition, natural ligands from other species which are devoid of lysines are not always available and ligands from other species may also exhibit reduced affinity or altered specificity for human receptors.

One strategy to circumvent these limitations is the generation of ligand variants devoid of side-chain reactive groups. Indeed, such an approach was recently applied to generate lysine-deficient TNF-α variants (Yamamoto et al., (2003) Nat. Biotechnol. 21:546-551). In this study the 6 lysine positions were randomized using the triplet NNS and TNF-α variants were selected against a TNF-α neutralizing antibody or TNF-RI. Interestingly, although two lysine residues were described to be vital for bioactivity, several of the TNF-α variants had other residues at these positions. One of lysine-deficient TNF-α variants with full biological activity could be specifically mono-PEGylated at its N-terminus leading to improved antitumor activity compared to randomly mono-PEGylated wild-tpye TNF-α. This study clearly demonstrates the usefulness of lysine-deficient ligands for site-directed modifications to improve therapeutic efficacy and further shows that phage display represents a powerful tool to isolate novel biologically active proteins with the desired properties.

However, in this approach the lysine positions were randomized with codons encoding all 20 amino acids, including lysine residues. The usage of triplets which do not encode for lysines should direct selection towards the enrichment of active ligands devoid of lysine residue, i.e. facilitate the enrichment of lysine-deficient active ligands. The present inventors, therefore, developed a novel approach using libraries of ligands with a biased codon usage at the triplets encoding lysine positions in the original sequence.

The advantage of this approach is that, (i) active peptides or polypeptides are selected/screened from a large combinatorial library of variants thus allowing identification from a large pool of possible sequences, (ii) finding optimal peptides or polypeptides sequences in respect to binding (and activity) which are devoid of those residues interfering with chemical coupling to other compounds, (iii) forcing isolation of peptides or polypeptides variants devoid of the interfering amino acid(s) by the use of a biased amino acid compositions.

Epidermal growth factor (EGF) is a monomeric protein consisting of 53 residues which binds specifically and with high affinity to the EGF receptor (Carpenter & Cohen, (1990) J. Biol. Chem. 265:7709-7712; Lemmon et al., (1997) EMBO J. 16:281-294). The EGF receptor is an attractive target for tumor therapy as it is overexpressed by a wide variety of human carcinomas, including cancers of the lung, liver, breast, head, neck, ovary, and bladder. Various conjugates consisting of EGF and cytotoxic drugs, ribonuclease, *Pseudomonas* exotoxin A, or radionuclides already demonstrated increased and selective delivery of the drugs into EGFR-expressing cells and the potential to inhibit tumor growth in animal models (Lutsenko et al., (2002) J. Drug Target. 10:567-571; Jinno et al., (1996) Cancer Chemother. Pharmacol. 38:303-308; Lee et al., (1993) Protein Eng. 6:433-440; Chen et al., (2002) Nucl. Med. Biol. 29:693-699). Furthermore, liposomes and viral vectors displaying EGF on their surface were developed for target cell-specific drug or gene delivery (Kullberg et al., (2002) Bioconjug. Chem. 13:737-743; Kullberg et al., (2003) Pharm. Res. 20:229-236; Kikuchi et al., (1996) Biochem. Biophys. Res. Comm. 227:666-671). In addition, targeting of drugs or toxins to EGF receptor-expressing tumor cells was also described for anti-EGFR antibody fusion proteins or conjugates (Aboud-Pirak et al., (1989) Proc. Natl. Acad. Sci. USA 86:3778-3781).

In this study we explored the possibility to generate EGF variants devoid of the 2 lysine residues present in human EGF. Since the guanidinium group of arginine may also serve as target for amino-reactive coupling groups under certain conditions we included mutagenesis of the 3 arginine residues of human EGF. Using a limited set of codons, avoiding lysine-encoding triplets but allowing arginine-encoding triplets, we were able to identify a panel of EGF variants lacking all lysine residues and containing only one arginine residue at the original position 41. One isolated EGF variant (EGFm1) with K28Q, R45S, K48S, and R53S mutations was expressed in bacteria and showed an identical binding activity as wild-type EGF. EGFm1 could be labeled with fluorescein-isothiocyanate demonstrating the accessibility of the N-terminal amino group for coupling reagents. Furthermore, coupling of EGFm1 to PEGylated liposomes resulted in target cell-specific binding and internalization of the liposomes. Such EGF variants are advantageous for directional and optimized chemical coupling to carrier systems or other molecules for the generation of anticancer therapeutics targeting the EGF receptor, which is overexpressed by a wide variety of different tumors.

Thus, one aspect of the present invention is a polynucleotide selected from the group consisting of
(a) polynucleotides encoding at least the mature modified epidermal growth factor (EGF) having the deduced amino acid sequence as shown in one of SEQ ID NOs 1-15;
(b) polynucleotides having the coding sequence, as shown in one of SEQ ID NOs: 16-30 encoding at least the mature modified EGF;
(c) polynucleotides encoding a fragment or derivative of a mature modified EGF encoded by a polynucleotide of any one of (a) to (b), wherein in said derivative one or more amino acid residues are conservatively substituted compared to said mature modified EGF with the proviso that polypeptide positions 28 and 48 are not Lys, and said fragment or derivative has epidermal growth factor receptor (EGFR) binding activity;
(d) polynucleotides, which are at least 50% identical to a polynucleotide as defined in any one of (a) to (c) and which code for a modified EGF having EGFR binding activity; and
(e) polynucleotides the complementary strand of which hybridizes, preferably under stringent conditions to a polynucleotide as defined in any one of (a) to (d) and which code for a modified EGF having EGFR binding activity;
or the complementary strand of such a polynucleotide.

A modified EGF having EGFR binding activity is a polypeptide that has at least 10% (e.g., at least: 10%, 20%; 30%; 40%; 50%; 60%; 70%; 80%; 90%; 95%; 98%; 99%; 99.5%; or 100% or even more) of the ability of the full-length wild type EGF to bind to EGFR. It is preferred that the modified EGF has at least wild type binding activity. Binding assays for assessing the binding of EGF to their respective receptors are well known in the art and include isothermal titration calorimetry (Lemmon et al. (1997) EMBO J. 16, 281-294), titration or competetion experiments with radiolabeled EGF (Campion et al. (1993) J. Biol. Chem. 268, 1742-1748), and surface plasmon resonance measurements (Lenferink et al. (2000) J. Biol. Chem. 275, 26748-26753) and further methods are also described herein below.

The modified EGF nucleic acid molecules of the invention can be DNA, cDNA, genomic DNA, synthetic DNA, or, RNA, and can be double-stranded or single-stranded, the sense and/or an antisense strand. Segments of these molecules are also considered within the scope of the invention, and can be produced by, for example, the polymerase chain reaction (PCR) or generated by treatment with one or more restriction endonucleases. A ribonucleic acid (RNA) molecule can be produced by *in vitro* transcription.

The polynucleotide molecules of the invention can contain naturally occurring sequences, or sequences that differ from those that occur naturally, but, due to the degeneracy of the genetic code, encode the same polypeptide, i.e. the polypeptides with SEQ ID NOs: 1 to 15. The polynucleotide can comprise additional polynucleotides at its 3' and/or 5' terminal end, which code for further polypeptides. The combined polynucleotides or fusion polynucleotides then encode a modified EGF fusion polypeptide. In a preferred embodiment the polynucleotide added 3' or 5' terminally will not comprise (a) codon(s) AAG and AAA encoding Lys and even more preferably will not comprise (a) codon(s) encoding AAA and AAG, encoding Lys and will have a reduced number of (a) codon(s) CGT, CGC, CGA, CGG, AGA and AGG encoding Arg.

The polynucleotide of fusion polynucleotide molecules of the invention can be synthesized *in vitro* (for example, by phosphoramidite-based synthesis) or obtained from a cell, such as the cell of a bacteria or mammal.

Polynucleotides encoding modified EGF disclosed herein can be identified based on its similarity to the sequences set forth in SEQ ID No. 16 to 30. For example, the identification can be based on sequence identity. In certain preferred embodiments the invention features isolated nucleic acid molecules which are at least 50% (or 55%, 65%, 75%, 85°/a, 95%, or 98%) identical to: (a) a nucleic acid molecule that encodes the polypeptide of SEQ ID NOs: 1 to 15; (b) the nucleotide sequence of SEQ ID NOs: 16-30; and (c) a nucleic acid molecule which includes a segment of at least 30 (e.g., at least 30, 40, 50, 60, 80, 100, 120, 140, or 159) nucleotides of SEQ ID NOs: 16 to 30 and code for a modified EGF having EGFR binding activity.

The determination of percent identity between two sequences is accomplished using the mathematical algorithm of Karlin and Altschul, *Proc. Natl. Acad. Sci. USA* **90**, 5873-5877, 1993. Such an algorithm is incorporated into the BLASTN and BLASTP programs of Altschul et al. (1990) J. Mol. Biol. 215, 403-410. To obtain gapped alignments for comparative purposes, Gapped BLAST is utilized as described in Altschul et al. (1997) Nucleic Acids Res. 25, 3389-3402. When utilizing BLAST and Gapped BLAST programs, the default parameters of the respective programs are used.

Hybridization can also be used as a measure of homology between two nucleic acid sequences. A nucleic acid sequence encoding a modified EGF as disclosed herein, or a portion thereof, can be used as a hybridization probe according to standard hybridization techniques. Hybridization conditions are known to those skilled in the art and can be found in Current Protocols in Molecular Biology, John Wiley & Sons, N.Y., 6.3.1-6.3.6, 1991. Moderate hybridization conditions are defined as equivalent to hybridization in 2X sodium chloride/sodium citrate (SSC) at 30°C, followed by a wash in 1 X SSC, 0.1% SDS at 50°C. Highly stringent conditions are defined as equivalent to hybridization in 6X sodium chloride/sodium citrate (SSC) at 45°C, followed by a wash in 0.2 X SSC, 0.1 % SDS at 65°C.

A further aspect of the present invention is a vector containing the polynucleotide(s) of fusion polynucleotide(s)of the present invention or a protein encoded by a polynucleotide or fusion polynucleotide of the present invention The term "vector" refers to a protein or a polynucleotide or a mixture thereof which is capable of being introduced or of introducing the proteins and/or nucleic acid comprised into a cell. It is preferred that the proteins encoded by the introduced polynucleotide are expressed within the cell upon introduction of the vector.

In a preferred embodiment the vector of the present invention comprises plasmids, phagemids, phages, cosmids, artificial mammalian chromosomes, knock-out or knock-in constructs, viruses, in particular adenoviruses, vaccinia viruses, attenuated vaccinia viruses, canary pox viruses, lentivirus (Chang, L.J. and Gay, E.E. (20001) Curr. Gene Therap. 1:237-251), herpes viruses, in particular Herpes simplex virus (HSV-1, Carlezon, W.A. et al. (2000) Crit. Rev. Neurobiol.), baculovirus, retrovirus, adeno-associated-virus (AAV, Carter, P.J. and Samulski, R.J. (2000) J. Mol. Med. 6:17-27), rhinovirus, human immune deficiency virus (HIV), filovirus and engineered versions thereof (see, for example, Cobinger G. P. et al (2001) Nat. Biotechnol. 19:225-30), virosomes, "naked" DNA liposomes, and nucleic acid coated particles, in particular gold spheres. Particularly preferred are viral vectors like adenoviral vectors or retroviral vectors (Lindemann et al. (1997) Mol. Med. 3:466-76 and Springer et al. (1998) Mol. Cell. 2:549-58). Liposomes are usually small unilamellar or multilamellar vesicles made of cationic, neutral and/or anionic lipids, for example, by ultrasound treatment of liposomal suspensions. The DNA can, for example, be ionically bound to the surface of the liposomes or internally enclosed in the liposome. Suitable lipid mixtures are known in the art and comprise, for example, DOTMA (1, 2-Dioleyloxpropyl-3-trimethylammoniumbromide) and DPOE (Dioleoylphosphatidyl-ethanolamine) which both have been used on a variety of cell lines.

Nucleic acid coated particles are another means for the introduction of nucleic acids into cells using so called "gene guns", which allow the mechanical introduction of particles into the cells. Preferably the particles itself are inert, and therefore, are in a preferred embodiment made out of gold spheres.

In a further aspect the polynucleotide or fusion polynucleotide of the present invention is operatively linked to expression control sequences allowing expression in prokaryotic and/or eukaryotic host cells. The transcriptional/translational regulatory elements referred to above include but are not limited to inducible and non-inducible, constitutive, cell cycle regulated, metabolically regulated promoters, enhancers, operators, silencers, repressors and other elements that are known to those skilled in the art and that drive or otherwise regulate gene expression. Such regulatory elements include but are not limited to regulatory elements directing constitutive expression like, for example, promoters transcribed by RNA polymerase III like , e.g., promoters for the snRNA U6 or scRNA 7SK gene, the cytomegalovirus hCMV immediate early gene, the early or late promoters of SV40 adenovirus, viral promoter and activator sequences derived from, e.g. , NBV, HCV, HSV, HPV, EBV, HTLV, MMTV or HIV; which allow inducible expression like, for example, CUP-1 promoter, the tet-repressor as employed, for example, in the tet-on or tet-off systems, the lac system, the trp system; regulatory elements directing cell cycle specific expression like, for example, cdc2, cdc25C or cyclin A; or the TAC system, the TRC system, the major operator and promoter regions of phage A, the control regions of fd coat protein, the promoter for 3-phosphoglycerate kinase, the promoters of acid phosphatase, and the promoters of the yeast α- or a-mating factors.

As used herein, "operatively linked" means incorporated into a genetic construct so that expression control sequences effectively control expression of a coding sequence of interest.

Another aspect of the present invention is a host cell genetically engineered with the polynucleotide or the fusion polynucleotide or the vector as outlined above. The host cells that may be used for purposes of the invention include but are not limited to prokaryotic cells such as bacteria (for example, *E. coli* and *B. subtilis*), which can be transformed with, for example, recombinant bacteriophage DNA, plasmid DNA, or cosmid DNA expression vectors containing the polynucleotide molecules of the invention; simple eukaryotic cells like yeast (for example, *Saccharomyces* and *Pichia*), which can be transformed with, for example, recombinant yeast expression vectors containing the polynucleotide molecule of the invention; insect cell systems like, for example, Sf9 of Hi5 cells, which can be infected with, for example, recombinant virus expression vectors (for example, baculovirus) containing the polynucleotide molecules of the invention; Xenopus oocytes, which can be injected with, for example, plasmids; plant cell systems, which can be infected with, for example, recombinant virus expression vectors (for example, cauliflower mosaic virus (CaMV) or tobacco mosaic virus (TMV)) or transformed with recombinant plasmid expression vectors (for example, Ti plasmid) containing a modified EGF encoding nucleotide sequence; or mammalian cell systems (for example, COS, CHO, BHK, HEK293, VERO, HeLa, MDCK, Wi38, and NIH 3T3 cells), which can be transformed with recombinant expression constructs containing, for example, promoters derived, for example, from the genome of mammalian cells (for example, the metallothionein promoter) from mammalian viruses (for example, the adenovirus late promoter and the vaccinia virus 7.5K promoter) or from bacterial cells (for example, the tet-repressor binding its employed in the tet-on and tet-off systems). Also useful as host cells are primary or secondary cells obtained directly from a mammal and transfected with a plasmid vector or infected with a viral vector. Depending on the host cell and the respective vector used to introduce the polynucleotide of the invention the polynucleotide can integrate, for example, into the chromosome or the mitochondrial DNA or can be maintained extrachromosomally like, for example, episomally or can be only transiently comprised in the cells.

A further aspect of the present invention is a transgenic non-human animal containing a polynucleotide, a fusion polynucleotide, a vector and/or a host cell as described above. The animal can be a mosaic animal, which means that only part of the cells making up the body comprise polynucleotides, vectors, and/or cells of the present invention or the animal can be a transgenic animal which means that all cells of the animal comprise the polynucleotides and/or vectors of the present invention or are derived from a cell of the present invention. Mosaic or transgenic animals can be either homo- or heterozygous with respect to the polynucleotides of the present invention contained in the cell. In a preferred embodiment the transgenic animals are either homo- or heterozygous knock-out or knock-in animals with respect to the genes which code for the proteins of the present invention. The animals can in principal be any animal, preferably, however, it is a mammal, selected from the group of non-human pimate horse, bovine, sheep, goat, pig, dog, cat, goat, rabbit, mouse, rat, guinea pig, hamster, or gerbil.

Another aspect of the present invention is a process for producing a modified EGF encoded by a polynucleotide of the present invention comprising: culturing the host cell described above and recovering the polypeptide encoded by said polynucleotide. Preferred combinations of host cells and vectors are outlined above and further combination will be readily apparent to someone of skill in the art. Depending on the intended later use of the recovered peptide a suitable cell type can be chosen. Eukaryotic cells are preferably chosen, if it is desired that the proteins produced by the cells exhibit an essentially natural pattern of glycosylation and prokaryotic cells are chosen, if, for example, glycosylation or other modifications, which are normally introduced into proteins only in eukaryotic cells, are not desired or not needed.

A further aspect of the invention is a process for producing cells capable of expressing modified EGF polypeptide comprising genetically engineering cells *in vitro* with at least one of the vectors described above, wherein said modified EGF polypeptide(s) is(are) encoded by a polynucleotide of the present invention.

Another aspect of the invention is a modified EGF having the amino acid sequence encoded by a polynucleotide of the invention or obtainable by the process mentioned above. The polypeptides of the invention include all those disclosed herein and functional fragments of these polypeptides. "Polypeptide" and "protein" are used interchangeably and mean any peptide-linked chain of amino acids, regardless of length or posttranslational modification. As used herein, a functional fragment of a modified EGF is a fragment of the modified EGF that is shorter than 53 amino acids but that has at least 10% (e.g., at least: 10%, 20%; 30%; 40%; 50%; 60%; 70%; 80%; 90%; 95%; 98%; 99%; 99.5%; or 100% or even more) of the ability of the full-length modified EGF to bind to EGFR. Binding assays are well know in the art as outlined above and are also described herein.

The polypeptides can be any of those described above but with not more than 20 (e.g., not more than: 20, 19, 18, 17, 16, 15, 14, 13, 12, 11, 10, nine, eight, seven, six, five, four, three, two, or one) conservative substitutions. Conservative substitutions typically include substitutions within the following groups: glycine and alanine; valine, isoleucine, and leucine; aspartic acid and glutamic acid; asparagine, glutamine, serine and threonine; lysine, histidine and arginine; and phenylalanine and tyrosine. All that is required of a polypeptide having one or more conservative substitutions is that it has at least 10% (e.g., at least: 10%, 20%; 30%; 40%; 50%; 60%; 70%; 80%; 90%; 95%; 98%; 99%; 99.5%; or 100% or even more) of the ability of the wild-type, full-length EGF to bind to EGFR.

In another preferred embodiment of the present invention the polypeptide or fusion polypeptide is coupled to at least one chemical moiety. Preferably the coupling is carried out via the N-terminal amino group of the modified EGF. The term "coupled" as used through out this specification means a direct or indirect covalent bond, between a polypeptide or fusion polypeptide of the present invention and another chemical entity. Indirect coupling is referred to, when rather than forming a direct covalent bond between the chemical entity and the polypeptide or fusion polypeptide a bi- or polyspecific coupling agent is used, which is capable of reacting with both the polypeptide or fusionpolypeptide of the present invention, preferably the N-terminal amino group and a residue in the chemical moiety. Examples of such coupling agents are but are not limited to p-Azidobenzoyl hydrate, 3-[2-Aminoethyl)dithio]propionic acid, N-[α.Maleimidoacettoxy]succinimide ester, N-5-Azido-nitrobenzoyloxN-5-Azidonitrobenzoyloxysuccinimide, N-[4-(p-Azidosalicylamido)butyl]3'-{2'-pyrixysuccinimide, N-[4-(p-Azidosalicylamido)butyl]3'-{2'-pyridyldithio}propionamide, p-Azidophenyl glyoxal monohydrate, 4-[p-Azidosalicylamido]butylamine, Bis-[β-(4-Azidosalicylamido)-ethyl]disulfide, 1,4-Bis-Maleimidobutane, 1,4-Bis-Maleimidyl-2,3-dihydroxybutane, Bis-Maleimidohexane, Bis-Maleimidoethane, N-β-Maleimidopropionic acid, N-[β-Maleimidopropionic acid] hydrazide-TFA, N-[β-Maleimidopropyloxy] succinimide ester, 1.8-Bis-Maleimidotriethyleneglycol, 1,11-Bis-Maleimidotetraethyleneglycol, Bis[2-(Succinimidyloxycarbonyloxy)ethyl]sulfone, Bis(Sulfosuccinimidyl)suberate, DCC, 1,5-Difluoro-2,4-dinitrobenzene, Dimethyl adipimidate, Dimethyl pimelimidate, Dimethyl suberimidate, 1,4-Di-[3'-(2'-pyridyldithio)-propionamido]butane, Disuccinimidyl glutarate, Dithiobis%succinimidyl propionate], 1-Ethyl-3-[3-dimethylaminopropyl]carbodiimide, Ethylene glycol bis[succinimidylsuccinate], N-s-Maleimidocaproic acid, N-[ε-Maleimidocaproic acid]hydrazide, N-[e-Maleimidocaproyloxy]succinimide ester, N-[y-Maleimidobutyryloxy]-succinimide ester, 1,6-Hexane-bis-vinylsulfone, N-κ-Maleimidoundecanoic acid, N-[κ-Maleimidoundecanoic acid]hydrazide, Succinimidyl-4-[N-maleimidomethyl]cyclohexane-1-carboxy-[6-amidocaproate], Succinimidyl 6-[3-(2-pyridyldithio}-propionamido]hexanoate, m-Maleimidobenzoyl-N-hydroxylsuccinimide, 4-[4-N-Maleimidophenyl]butyric acid hydrazide, Methyl N-succinimidyl adipate, N-Hydroxysuccinimidyl-4-azidosalicylic acid, 3-[2-Pyridyldithio]propionyl hydrazide, N-[p-Maleimidophenyl]isocyanate, -Succinimidyl [4-azidophenyl] 1,3'-dithiopropionate, Sulfosuccinimidyl 2-[7-azido-4-methyl-coumarin-3-acetamido]ethyl-1,3'-dithiopropionate, Sulfosuccinimidyl 2-[m-azido-o-nitrobenzamido]ethyl-1,3'dithiopropionate, N-Succinimidyl 6-[4'-azido-2'-nitrophenylamino]hexanoate, Sulfosuccinimidyl 2-[p-azido-salicylamido]ethyl-1,3'-dithiopropionate, N-Succinimidyl S-acetylthioacetate, N-Succinimidyl S-acetylthio-propionate, Succinimidyl 3-[bromoacetamido]propionate, Sulfosuccinimidyl-[perfluoroazido-benzamido]-ethyl-1,3'dithiopropionate, N-Succinimidyl iodacetate, N-Succinimidyl [4-iodacetyl]aminobenzoate, Succinimidyl 4-[N-maleimidomethyl]cyclohexane-1-carboxylate, Succinimidyl 4-[p-maleimidophenyl]butyrate, Succinimidyl-6-[β-maleimidopropionamido]hexanoate, 4-Succinimidyloxycarbonyl-methyl-α[2-pyridyldithio]toluene, Succinimidyl-[4-psoralen-8-yloxy]-butyrate, N-Succinimidyl 3-[pyridyldithio]propionate, Disulfosuccinimidyl tartrate, Ethylene glycol bis[sulfosuccinimidylsuccinate, N-[ε-Maleimidocaproyloxy]sulfosuccinimide ester, N-[γ-Maleimidobutyryloxy]sulfo-succinimide ester, N-Hydroxysulfosuccinimidyl-4-azidobenzoate, N-[κ-Maleimidoundecanoyloxy]sulfosuccinmide ester, Sulfosuccinimidyl-6-[α-methyl-α-(2-pyridyldithio)-toluamido]-hexanoate, Sulfosuccinimidyl-6-[3-(pyridyl dthio)propionamido]hexanoate, m-Maleimidobenzoyl-N-hydroxysulfo-succinimide ester, Sulfosuccinimidyl[4-azidosalicylamido]-hexanoate, Sulfosuccinimdyl[4-azidophenyldithio]propionate, Sulfosuccinimidyl 6-[4'-azido-2'-nitrophenylamino]hexanoate, Sulfosuccinimidyl[4-iodoacetyl]aminobenzoate, Sulfosuccinimidyl 4-[N-maleimidomethyl]cyclohexane-l-carboxylate, Sulfosuccinimidyl 4-[p-maleimidophenyl]butyrate, N-[ε-Trifluoroacetylcaproyloxy]succinimide ester, Sulfosuccinimidyl [2-6-(biotinamido) 2-(p-azidobenzamido)-hexanoamido]ethyl-1,3'-dithiopropionate, Tris-succinimidyl aminotriacetate, β-[Tris-(hydroxymethyl)phosphino]propionic acid, Tris-[2-maleimidoethyl]amine, and aldehyde-activated dextrane.

The term "chemical moiety" or "entity" as used interchangeably herein is not limited to a particular type of a chemical substances, however, in a preferred embodiment the chemical moiety is selected from the group consisting of a spacer, a marker, a tag, and a lipid and in particular a phospholipid, a drug, a capping group, a polypeptide and a spacer attached to a second chemical moiety.

Any naturally or non-naturally occurring polypeptide can be coupled to the modified EGF or fusion polypeptide thereof, however in a preferred embodiment the polypeptide is selected from the group consisting of a cytokine, a chemokine, a growth factor, an adhesion molecule, an antibody light and/or heavy chain, a single chain antibody, a toxin, an enzyme, a receptor ligand, a lytic peptide, a membrane insertion sequence and a fluorescent protein or fragments thereof.

A "capping group" within the meaning of the present invention is a chemical moiety which protects the molecule to which it is attached from, for example, chemical or enzymatic degradation. The capping group or groups can be attached directly to the polypeptide of the present invention or to any other chemical moiety, which is itself attached to the polypeptide of the present invention. In certain preferred aspects of the present invention in which the N- and/ or C-terminus of the polypeptide of the present invention is not attached to another chemical moiety like, for example, a spacer and would otherwise be in its "free" form, i.e. -COOH and/or -NH₂, a capping group is attached to one or both ends to avoid or minimize degradation by, for example, exoproteinases or the like.

The capping groups of the present invention have in a preferred embodiment one of the following structures:

R―A

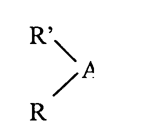

Wherein A is an amino acid or an amino acid residue mimetic of the polypeptide of the present invention or any other chemical moiety attached to the polypeptide of the present invention and R and R' each independently have the meaning: -C(O)R₁, -C(O)NHR₁, -S(O)2R1, -C(O)OR₁, CR₁ or R₁,
wherein R₁ can have the meaning H; linear or branched alkyl, in particular lower alkyl (C₁, C₂, C₃, C₄, and C₅, e.g. methyl, ethyl, n-propyl, isopropyl, n-butyl, isobutyl, tert-butyl, n-pentyl or iso-pentyl); substituted linear or branched alkyl, in particular lower substituted alkyl; linear or branched alkenyl, in particular lower alkenyl (C₂, C₃, C₄ and C_{5,}, e.g. ethenyl, 1-propenyl, 2-propenyl, iso-propenyl, 1-butenyl, 2-butenyl, 3-butenyl; substituted linear or branched alkenyl, in particular lower substituted alkenyl; linear or branched alkynyl, in particular lower alkynyl (C₂, C₃, C₄ and C₅); substituted linear or branched alkynyl, in particular lower substituted alkynyl; linear or branched alkanol, in particular lower alkanol (C₁, C₂, C₃, C₄, and C₅); linear or branched alkanal, in particular lower alkanal (C₁, C₂, C₃, C₄, and C₅, e.g. COH, CH₂COH, CH₂CH₂COH; aryl, in particular phenyl; substituted aryl, in particular substituted aryl; haloalkyl, in particular lower haloalkyl (C₁, C₂, C₃, C₄, and C₅); haloalkoxy, in particular lower haloalkoxy (C₁, C₂, C₃, C₄, and C₅); heteroaryl optionally comprising 1 to 4 heteroatoms selected from N, O, S; substituted heteroaryl optionally comprising 1 to 4 heteroatoms selected from N, O, S; aryl, in particular C₅ to C₁₂); alkylaryl, in particular C₅ to C₁₂, e.g. benzyl, isoquinolinyl, quinolinyl, naphthyl; substituted alkylaryl, in particular C₅ to C₁₂, e.g. substituted benzyl; alkylheteroaryl optionally comprising 1 to 4 heteroatoms selected from N, O, S optionally comprising 1 to 4 heteroatoms selected from N, O, S; substituted alkylheteroaryl optionally comprising 1 to 4 heteroatoms selected from N, O, S; aminoalkyl, in particular C₁, C₂, C₃, C₄ and C₅, e.g. -NHCH₃, -NHCH₂CH3, -N(CH₃)₂; substituted aminoalkyl; dialkylamino; aminoketone, in particular -NHCOCH₃; substituted aminoketone; aminoaryl, in particular -NH-Ph; substituted aminoaryl, in particular substituted -NH-Ph; carboxyl, carboalkoxy; alkylcarboxamide; cycloalkyl; alkylcycloalkyl; CN; NH₂; Halogen, in particular F, Cl, and Br; if the residues mentioned above are substituted they are preferably mono, di, or tri substituted with a substituent selected from the group of halogen, in particular F, Cl, and Br, NH₂, NO₂, OH, SH, NH, CN, aryl, alkylaryl, heteroaryl, akylheteroaryl, COH or COOH;
R and R' form a ring comprising 5-7 atoms selected from C, N, S and O; or
R and R' are independently toluenesulfonyl, methanesulfonyl, FMOC or (+)-menthyloxy-CO-

The term "spacer" refers to a chemical moiety, which serves the purpose of providing the accessibility of the modified EGF even when attached to other chemical moieties which might otherwise sterically hinder the binding of the modified EGF or fusion polypeptides thereof to its target structures. Spacer within the meaning of the present invention are a linear extension of at least 0.5 nm preferably the spacer has a linear extension of between 1 and 10 nm and even more preferably between 2 and 5 nm. The spacer is preferably a linear or branched saturated or unsaturated carbohydrate chain. The carbohydrate chain preferably comprises multimeric repeats of a monomeric building block. Depending on the length of the respective monomeric building block between 2 and 10 multimeric repeats of the monomeric building blocks are preferred. In preferred embodiments the spacer is hydrophilic. The spacer can comprise a functional group which allows attachment to the polypeptides of the present invention on one terminus and another functional group on the other terminus, which allows attachment of the spacer to another chemical moiety.

Preferred spacers are bifunctional molecules in particular bifunctional polyethylene or polypropylene glycol derivatives comprising preferably between about 1 and 40 repeat units, oligopeptides comprising natural and/or synthetic amino acids and preferably between 1 to 40 preferably 2 to 20 and more preferably 2 to 10 amino acids. A particular preferred building block of a spacer of the present invention is 8-amino-3, 6-dioxatanoic acid (doo) and spacers comprising between 1 to 10 repeat units of doo are preferred. Spacers comprising between 2 and 5 doo units being more preferred and spacers comprising 3 doo units being most preferred. In the context of liposomes it has been discovered that there is an optimal length of the spacer, which is between 2 and 5 nm. While spacers with a length of less than 0.5 nm will in most cases not provide enough distance from, for example, the liposomal surface to which the polypeptides of the present invention has been attached to allow efficient interaction, i.e. binding, between the modified EGF or fusion polypeptide thereof and EGFR, bearing cells. On the other hand spacers, which are longer than 10 nm show an increasing "floppiness" which is also detrimental to the binding to cells.

The term "marker" refers to a chemical moiety which is directly or indirectly detectable by analytical methods including measurement of fluorescence, nuclear magnetic resonance, computer tomography or scintigrams and comprises without limitation electron dense molecules, paramagnetic molecules, superparamagnetic molecules, radioactive molecules like, for example, ¹³N, ¹⁵O, ¹⁸F, ⁵¹Gr, ⁵⁴Fe, ⁶⁰Co, ⁶⁷Ga, ⁷⁵Se, ^{99m}Tc, ¹¹¹In, ^{112m}Ag, ^{113m}In, ¹²³I, ¹³³Xe, ¹⁴⁸Au, ³⁵S, ³³P, ³²P, or ¹¹C, non-radioactive isotopes, which include, for example, ²H and ¹³C, and fluorescent molecules or molecules generating fluorescence or light emission like, for example, green fluorescent protein, luciferase, and a variety of fluorescent dies all of which are well known to someone of skill in the art.

The term "tag" refers to chemical moieties, which allow purification of the polypeptides or complexes comprising the polypeptides of the present invention like, for example, biotin, Chitin-tag, Myc-tag, His-tag or the like, which are all well known in the art.

In a preferred embodiment the modified EGF or fusion polypeptide thereof is attached directly or indirectly to a lipid. The type of lipid to which the polypeptide can be attached is not particular limited. However, in preferred embodiments the respective lipid can be inserted or incorporated into lipid-based carriers like, for example, liposomes or virosomes. Particularly suitable lipids are glycerides, glycerophospholipides, glycerophosphinolipids, glycerophosphonolipids, sulfolipids, sphingolipids, phospholipids, isoprenolides, steroids, stearines, steroles, and/or carbohydrate containing lipids. Particularly preferred lipids for the attachment of the polypeptides of the present invention are phospholipids preferably phosphatidylcholine (PC), phosphatidylserine (PS), and phosphatidylethanolamine (PE), in particular distearoylphosphatidyl (DSPE) or alpha-(dipalmitoylphosphatidyl (DPP)) which are often included in liposomes used for delivery of drugs.

In another embodiment the lipid attached to the modified EGF or fusion polypeptide thereof is selected from the group consisting of N-caproylamine-PE, N-dodecanylamine-PE, phophatidylthioethanol, N-[4-(p-maleimidomethyl)cyclohexane-carboxamide-PE (N-MCC-PE), N-[4-(p-maleimidophenyl)butyramide]-PE (N-MPB), N-[3-(2-pyridyldithio)propionate]-PE (N-PDP), N-succinyl-PE, N-glutaryl-PE, N-dodecanyl-PE, N-biotinyl-PE, N-biotinyl-cap-PE, phosphatidyl-(ehtylene glycol), PE-polyethylene glycol (PEG)-carboxylic acid, PE-PEG-maleimide, PE-PEG-PDP, PE-PEG-amine, PE-PEG-biotin, PE-PEG-HNS, dipalmitoyl-glycerosuccinyl-lysine, alpha-methoxy-omega-(1,2-dioctadecenoyloxy glyceryl) (DO), alpamethoxy-omega-(1,2-ditetradecenoyloxy glyceryl) (DT).

In a further preferred embodiment the modified EGF or fusion polypeptide thereof is attached to at least one drug. The term "drug" encompasses any therapeutically active compound and in particular compounds are selected from the group consisting of immunosuppressants, immunostimulants, antibiotics, antiinfectives, antiallergics, cytostatics, cytotoxic agents and prodrugs thereof, mitogens, chemokines, cytokines, dermatics and/or physiological or pharmacological inhibitors of mitogens, chemokines or cytokines. In preferred embodiments the drug is attached to the modified EGF or fusion polypeptide thereof in such a way that it is releasable and preferably the release of the drug is primarily effected in the tissues or areas of the body to which the polypeptide of the present invention binds i. e. primarily in tumor endothelium or tumors. Particularly preferred means of attachment of the drug are short polypeptide stretches which are cleavable, for example, by enzymes which are released at the target site. Thus, preferably the drugs are released in the tumor endothelium or in tumors. Enzymes of this type include, for example metalloproteinases. Such releasable connections are known in the art and can be selected to provide a further specificity on top of the specificity already achieved by the target specific binding of the polypeptides of the present invention. The inclusion of such a cleavage site is particularly desirable, in cases in which a drug attached to the polypeptides of the present invention exerts a cytotoxic and/or growth inhibitory effect on cells once it is released.

In a preferred embodiment of the present invention using above described spacer the spacer is attached at one side to the modified EGF or fusion polypeptide thereof and on the other side to a second chemical moiety. Preferably the second chemical moiety is selected from the group consisting of a drug, a marker, a tag and a lipid. In this context the terms "drug", "marker", "tag", "polypeptide" and "lipid", have the meaning and preferred meanings as outlined above.

The modified EGF or fusion polypeptide thereof can be used directly as a therapeutic or in combination with additional substances and, therefore, the present invention in a further aspect relates to a composition comprising at least one polypeptide of the present invention and at least one further component selected from the group consisting of liposomes, virosomes, microsphere, niosomes, dendrimeres, stabilizers, buffers, excipient, additives.

Liposomes are single or multilamellar lipid vesicles of varying size. The size is preferable between 10 and 1000 nm and more preferably between 50 and 500 nm and most preferably between 80 and 200 nm. Virosomes are liposomes with a lipid composition closely resembling the lipid composition of viruses and which in preferred embodiments have proteins integrated into the membrane (Kaneda (2000) Adv. Drug. Deliv. Rev. 43:197-205). Microspheres are spherical particle with large size (up to 2 mm) and rigid morphology containing a core substance (Ravi & Kumar (2000) J. Pharm. Sci. 3:234-258). Niosomes are non-ionic surfactant vesicles (Baillie et al. (1985) J. Pharm. Pharmacol. 37:863-868). Dendrimers are synthetic, highly branched, mono-disperse macromolecules of nanometer dimensions (Patri et al. (2002) Curr. Opin. Chem. Biol. 6:466-471). Buffers comprised in the composition of the present invention can be any physiological buffer substances including, for example phosphate buffer or Hepes.

Excipients which facilitate production and administration of the compositions of the present invention are the art known excipients and include, for example, alginates, calcium carbonate, dextrose, fructose, lactose, maltose, maltodextrin, and the like. Stabilizers are also known in the art and comprise, for example, α-tocopherol and various carbohydrates.

In a preferred embodiment of the compositions of the present invention the modified EGF or fusion polypeptide thereof is integrated into or attached to a liposome, virosome, microsphere, niosome or dendrimer, which allows the respective entity to be target to sites and tissues in the body expressing EGFR. Polypeptides can be attached to one of the components, which are used for the generation of liposomes, virosomes, microsphere, niosomes, or dendrimers prior, during or after formation of the respective structure. In particular for liposomes and virosomes it is envisioned that modified EGF or fusion polypeptide thereof which are linked to a lipid either with or without an intervening spacer as defined above are integrated into the lipid mono or multilayer of the liposome or virosome. In preferred embodiments the modified EGF or fusion polypeptide thereof is primarily comprised on the outer surface of the respective liposome or virosome to allow interaction of the modified EGF or fusion polypeptide thereof with EGFR, preferably upon administration of the composition of the present invention to a patient. In preferred embodiments the modified EGF or fusion polypeptide thereof of the present invention will be attached to between about 0.1 mol% to about 10 mol% of all components which are used for the generation of the respective structure. These ranges are particular preferred for liposomes and virosomes.

In preferred embodiments the liposomes or virosomes of the present invention comprise lipids selected from the group consisting of glycerides, glycerophospholipides, glycerophosphinolipids, glycerophosphonolipids, sulfolipids, sphingolipids, phospholipids, isoprenolides, steroid, stearines, steroles, and carbohydrate containing lipids. In preferred embodiments the liposome or virosome comprises cholesterol (CH) and sphingomyelin (SM). More preferably cholesterol is comprised in relation to the total molar lipid composition of the liposome or virosome at a molar ratio of about 40 to about 60 mol% and more preferably of about 45 to about 55 mol% and most preferably of about 48 to about 52 mol%. SM on the other hand is preferably present in relation to the total molar lipid composition of the liposome at a molar ratio of about 10 to about 20 mol% and more preferably of about 11 to 18 mol% and most preferably of about 12 to about 16 mol%. In a particular preferred embodiment the liposome or virosome comprises in relation to the total molar lipid composition CH and SM at a molar ration of about 40 to about 60 mol% and of about 10 to about 20 mol%, respectively, and even more preferred at a molar ratio of about 45 to about 55 mol% and of about 11 to about 18 mol%, respectively and most preferably of about 48 to about 52 mol% and of about 12 to about 16 mol%, respectively.

The liposomes or virosomes which are comprised in the composition to the present invention contain (a) further lipid(s). This(These) is(are) preferably selected from the above indicated preferred lipids. In preferred embodiments at least one of the additional lipids is selected from PE and PC. In a particular preferred embodiment PE is present in the liposome or virosome of the present invention and in particular, if CH and SM are also present in the liposome or virosome. Preferably CH and SM are present together with PE in the above indicated preferred and particularly preferred ranges. PE itself is present in preferred embodiments in relation to the total molar lipid composition of about 5 to about 25 mol%, preferably about 10 to about 20 mol% and most preferably about 12 to about 18 mol%. Again in particular preferred embodiments CH, SM, and PE are present in above indicated preferred or particular preferred ranges.

In a further embodiment the additional lipids comprise PE and PC and in a preferred embodiment PE and PC are present in relation to the total molar lipid composition of the liposome or virosome at a molar ratio of about 5 to about 25 mol% and about 15 to about 40 mol%, respectively. The above ranges for PE are PC particular preferred, if the molar ratio of CH and SM are as indicated above.

In a further embodiment any of the components making up the membrane of the liposomes of the present invention can be coupled or non-covalently attached to a further chemical moiety. The meaning of the term chemical moiety is as defined above. However, in preferred embodiments the chemical moiety is a stabilizing moiety. Stabilizing moieties within the meaning of this invention increase the circulation time of the liposome once it is administered. Particular preferred stabilizing moieties are ganglioside GM1, phosphatidylinositol or PEG, particular preferred PEGs have a molecular mass between about 1,000 and about 10,000 g/mol, more preferably about 5,000 g/mol.

In a preferred embodiment the chemical moieties and in particular the stabilizing moieties are coupled or attached to only a fraction of the molecules making up the membrane of the liposomes. It is preferred that between about 1 to about 20 mol% of the components of the liposomal membrane carry an attached chemical moiety, more preferably between about 3 and about 10 mol% and even more preferably about 5 mol%.

A preferred liposomal component for coupling or attachment of the chemical moiety, in particular for the stabilizing moiety is a lipid component. While different chemical moieties can be coupled or attached to different lipid components it is preferred that the chemical moiety(ies) is(are) coupled or attached to one or more of the phospholipids comprised within the liposome of the present invention. In a further preferred embodiment the one or more chemical moiety is coupled or attached to PE. In particular, if a stabilizing agent like, for example, PEG is used PE is used for attachment.

In addition to the coupling or attachment of stabilizing moieties detergents, proteins and peptides can be incorporated into the liposome for stabilizing the lipid bilayers of the liposomes of the present invention. Detergents which can be used as bilayer stabilizing components include, but are not limited to, Triton X-100, deoxycholate, octylglucoside and lysophosphatidylcholine. Proteins which can be used as bilayer stabilizing components include, but are not limited to, glycophorin and cytochrome oxidase. In preferred embodiments a liposome can comprise between 0.05 and 15 mol% of a stabilizing agent.

To exert a therapeutic effect the composition of the present invention can further comprise a drug. Preferably this drug is coupled or attached directly or indirectly to a modified EGF or fusion polypeptide thereof or comprised or attached to the liposomes, virosomes, microspheres, etc.

If a drug or diagnostic is comprised within a liposome it is particularly preferred that the drug or diagnostic is comprised in the interior of the liposome or in cases of lipophilic drugs also within or between the lipid bilayers. In preferred embodiments the drug is comprised within the liposome, virosome, microsome or niosome. A variety of methods are available in the prior art to "load" a liposome, virosome, microsome or niosome with a given drug or diagnostic. In its simplest form the drug and/or diagnostic is/are admixed with the lipid components during formation of the liposomes. Other passive loading methods include dehydration-rehydration (Kirby & Gregoriadis (1984) Biotechnology 2:979), reverse-phase evaporation (Szoka & Papahadjopoulos (1978) Proc. Natl.Acad. Sci. USA 75:4194-), or detergent-depeletion (Milsmann et al. (1978) Biochim. Biophys. Acta 512:147-155). However, these techniques often lead to a substantial loss of drug during loading, which is a particular disadvantage in cases where the drug is expensive.

Other methodologies for encapsulating drugs and/or diagnostics include so called "remote loading" or "active loading" in which due to a gradient, for example, a pH or salt gradient between the exterior and the interior of a preformed liposome the drug and/or diagnostic is transported into the liposome along the gradient (see, for example Cheung et al. (1998) Biochim. Biophys. Acta 1414:205-216; Cullis et al. (1991) Trends Biotechnol. 9:268-272; Mayer et al. (1986) Chem. Phys. Lipids 40:333-345).

The passive and active loading techniques referred to above and other methods well known in the art can all without limitation employed by the skilled artisan. The most efficient method of loading for any given drug or diagnostic can be determined by routine experimentations by well established procedures. Variables which are typically adjusted are pH, temperature, salt type and concentration, type of buffer etc.

In a preferred embodiment the drugs and/or diagnostics are loaded by remote loading into the liposomes, virosomes, microsomes or niosomes, since this method offers a very low loss of the substance to be loaded. In a preferred embodiment a pH gradient is used for loading. Depending on the substance to be loaded the interior of the liposome will typically be acidified with respect to its exterior. Preferably the interior will have a pH between 1 and 6 prior to loading with the drug or diagnostic.

Particularly preferred drugs are selected from the group consisting of analgesics, antirheumatics, anthelminthics, antiallergics, antianemics, antiarrhythmics, antibiotics, angiogenesis inhibitors, antiinfectives, antidemenics (nootropics), antidiabetics, antidotes, antiemetics, antivertiginosics,, antiepileptics, antihemorrhagics, antihypertonics, antihypotonics, anticoagulants, antimycotics, antitussiv agents, antiviral agents, beta-receptor and calcium channel antagonists, broncholytic and antiastmatic agent, chemokines, cytokines, mitogens, cytostatics, cytotoxic agents and prodrugs thereof, dermatics, hypnotics and sedatives, immunosuppressants, immunostimulants, peptide or protein drugs, in particular hormones and physiological or pharmacological inhibitors of mitogens, chemokines, or cytokines or their respective prodrugs. Of course it is also envisioned that a liposome of the invention comprises more than one drug at once. Numerous drugs for each category are known to the skilled artisan and are all included without limitation.

Since expression of EGFR is particularly found epithelial and stromal cells and even more particularly on tumor cells of lung cancer, liver cancer, head and neck cancer, bladder, cancer, prostate cancer, cervix cancer, endometrial cancer, colorectal adenoma and adenocarcinoma, gastric cancer, oesophageal cancer, breast cancer, squamous carcinoma, glioblastomas and other high-grade primary brain tumors (Nicholson et al. (2001) Eur. J. Cancer 37, S9-S15) the polypeptide of the present invention allow the specific targeting of drugs, which can interfere with tumor growth and/or progression of the disease in a targeted manner, therefore, the composition of the present invention are particular suitable for treatment of hyperproliferative diseases associated with neovascularization. Consequently, particular preferred drugs are cytostatics and cytotoxic drugs. A large variety of such drugs are known in the art. Particular preferred cytostatics and cytotoxic drugs are selected from the group consisting of alkylating substances, anti-metabolites, antibiotics, epothilones, nuclear receptor agonists and antagonists, anti-androgenes, anti-estrogens, platinum compounds, hormones and antihormones, interferons and inhibitors of cell cycle-dependent protein kinases (CDKs), inhibitors of cyclo-oxygenases and/or lipoxygenases, biogeneic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or their respective derivatives or analogs thereof. Several of the above indicated drugs are now administered simultaneously for cancer therapy and, consequently, it is also envisioned that more than one cytostatic and/or cytotoxic drug is comprised in a liposome of the present invention.

The term "immunosuppressant" comprises both substances which lower the activity of immune response as well as substances with an anti-inflammatory action, preferred examples are glucocorticoids, in particular beclomethasone, betamethasone, clocortolone, cloprednol, cortisone, dexamethasone, fludrocortisone, fludroxycortide, flumetasone, fluocinolone acetonide, fluocinonide, fluocortolone, fluorometholone, fluprednidene acetate, hydrocortisone, paramethasone, prednisolone, prednisone, prednylidene, pregnenolone, triamcinolone or triamcinolone acetonide, a cyclosporin, in particular cyclosporin A, mycophenolate mofetil, tacrolimus, rapamycin, FK 506, cycloheximide-N-(ethyl ethanoate), azathioprine, ganciclovir, an anti-lymphocyte globulin, ascomycin, myriocin, a pharmacological inhibitor of MAP kinases (especially a p38 inhibitor such as VX-745), caspase inhibitors, matrix metalloproteinase inhibitors, and/or methotrexate.

The term "immunostimulant" encompasses all substances, which influence the function of cells which are involved directly or indirectly in mediation of the immune response, and where the influence leads to an immune response. These cells include, for example, macrophages, Langerhans cells and other dendritic cells, lymphocytes, indeterminate cells, but also cells which do not themselves belong to the immune system but are involved in immune disorders of the skin, such as fibroblasts, keratinocytes and melanocytes, but especially Langerhans cells. The strength of the immune response can be determined for example through the amount of cytokines produced (such as interferon-gamma), detection of activation markers on dendritic cells (such as MHCII or CD86) or the number of activated CD8-positive T cells in the skin. Immunostimulants for the purpose of the present invention are, in particular, plant immunostimulants which are obtained, for example, from Echinacea pallida or Echinacea purpurea, cytokines such as, for example, interleukins, interferons and colony-stimulating factors, and bacterial constituents or molecules which mimic the latter [such as bacterial DNA and unmethylated oligodeoxynucleotides with CpG sequences, and constituents of the bacterial cell wall or coat, especially the lipopolysaccharides and molecules derived therefrom, such as monophosphoryl-lipid A, muramyldipeptide (N-acetylmuramyl-L-alanyl-D-isoglutamine), and/or PamCys3, and other molecules such as tetanus toxoid, poly-L-arginine or MHCII peptides].

The term "antibiotics" encompasses, for example, penicillins, cephalosporins, tetracyclines, aminoglycosides, macrolide antibiotics, lincosamides, gyrase inhibitors, sulfonamides, trimethoprim, polypeptide antibiotics, nitroimidazole derivatives, amphenicol, in particular actinomycin, alamethicin, alexidine, 6-aminopenicillanic acid, amoxicillin, amphotericin, ampicillin, anisomycin, antiamoebin, antimycin, aphidicolin, azidamfenicol, azidocillin, bacitracin, beclomethasone, benzathine, benzylpenicillin, bleomycin, bleomycin sulfate, calcium ionophore A23187, capreomycin, carbenicillin, cefacetrile, cefaclor, cefamandole nafate, cefazolin, cefalexin, cefaloglycin, cefaloridine, cefalotin, cefapirin, cefazolin, cefoperazone, ceftriaxone, cefuroxime, cephalexin, cephaloglycin, cephalothin, cephapirin, cerulenin, chloramphenicol, chlortetracycline, chloramphenicol diacetate, ciclacillin, clindamycin, chlormadinone acetate, chlorpheniramine, chromomycin A3, cinnarizine, ciprofloxacin, clotrimazole, cloxacillin, colistine methanesulfonate, cycloserine, deacetylanisomycin, demeclocycline, 4,4'-diaminodiphenyl sulfone, diaveridine, dicloxacillin, dihydrostreptomycin, dipyridamole, doxorubicin, doxycycline, epicillin, erythromycin, erythromycin stolate, erythromycin ethyl succinate, erythromycin stearate, ethambutol, flucloxacillin, fluocinolone acetonide, 5-fluorocytosine, filipin, formycin, fumaramidomycin, furaltadone, fusidic acid, geneticin, gentamycin, gentamycin sulfate, gliotoxin, gramicidin, griseofulvin, helvolic acid, hemolysin, hetacillin, kasugamycin, kanamycin (A), lasalocid, lincomycin, magnesidin, melphalan, metacycline, meticillin, mevinolin, micamycin, mithramycin, mithramycin A, mithramycin complex, mitomycin, minocycline, mycophenolic acid, myxothiazole, natamycin, nafcillin, neomycin, neomycin sulfate, 5-nitro-2-furaldehyde semicarbazone, novobiocin, nystatin, oleandomycin, oleandomycin phosphate, oxacihin, oxytetracycline, paromomycin, penicillin, pecilocin, pheneticillin, phenoxymethylpenicillin, phenyl aminosalicylate, phleomycin, pivampicillin, polymyxin B, propicillin, puromycin, puromycin aminonucleoside, puromycin aminonucleoside 5'-monophosphate, pyridinol carbamate, rolitetracycline, rifampicin, rifamycin B, rifamycin SV, spectinomycin, spiramycin, streptomycin, streptomycin sulfate, sulfabenzamide, sulfadimethoxine, sulfamethizole, sulfamethoxazole, tetracycline, thiamphenicol, tobramycin, troleandomycin, tunicamycin, tunicamycin A1 homolog, tunicamycin A2 homolog, valinomycin, vancomycin, vinomycin A1, virginiamycin M1, viomycin and/or xylostasin.

The term "antiinfectives" encompasses, for example, antimycotics, agents with antiparasitic effect and virustatics, in particular amphotericin, vifonazole, buclosamide, quinoline sulfate, chlormidazole, chlorphenesin, chlorquinaldol, clodantoin, cloxiquine, cyclopirox olamine, dequalinium chloride, dimazole, fenticlor, flucytosine, griseofulvin, ketoconazole, miconazole, natamycin, sulbentine, tioconazole, toinaftate, antiretroviral agents and/or herpes remedies.

The term "antiallergics" encompasses, for example, substances from the class of globulins, corticoids or antihistamines, in particular beclomethasone and derivatives thereof, betamethasone cortisone and derivatives thereof, dexamethasone and derivatives thereof, bamipine acetate, buclizine, clemastine, clemizole, cromoglicic acid, cyproheptadine, diflucortolone valerate, dimetotiazine, diphenhydramine, diphenylpyraline, ephedrine, fluocinolone, histapyrrodine, isothipendyl, methdilazine, oxomemazine, paramethasone, prednylidene, theophylline, and/or tolpropamine tritoqualine.

The term "mitogens", "chemokines" and "cytokines" encompass, for example, interferon-alpha, interferon-beta, interferon-gamma, interleukin-1, interleukin-2, interleukin-7, interleukin-10, interleukin-12, interleukin-18, GM-CSF, MIP-1-alpha/beta, RANTES, EGF, basic or acidic FGF, PDGF, IGF, VEGF, TGF-beta and/or TNF-alpha.

The term "dermatics" encompasses, for example, shale oil sulfonates, tar and tar derivatives, astringents, antihidrotics, acne remedies, antipsoriatics, antiseborrheic agents and/or enzyme preparations for the treatment of skin defects.

Consequently, a further aspect of the invention is the use of the modified EGF or fusion polypeptide thereof or a polynucleotide, a vector or a cell or a composition of the invention for the production of a medicament for the therapy of a proliferative disease or a disease in which cells in or adjacent a disease site show an altered expression and in particular an overexpression of EGFR, if compared to healthy or normal tissue.

Since the expression or over expression of EGFR has been reported in particular for proliferative diseases this type of diseases are preferred diseases which can be treated with the modified EGF or fusion polypeptide thereof, a polynucleotide encoding these, a vector, a cell or a composition of the present invention. Particular preferred proliferative diseases are selected from the group consisting of lung cancer, liver cancer, head and neck cancer, bladder, cancer, prostate cancer, cervix cancer, endometrial cancer, colorectal adenoma and adenocarcinoma, gastric cancer, oesophageal cancer, breast cancer, squamous carcinoma, glioblastomas and other high-grade primary brain tumors, chronic inflammatory proliferative diseases, vascular proliferative diseases and virus-induced proliferative diseases.

Furthermore the modified EGF or fusion polypeptide thereof and composition of the present invention can be used for diagnostic purposes in particular, if they are attached to a marker as defined above. Therefore, another aspect of the present inventions is the use of a polypeptide or composition of the present invention for the diagnosis of a disease and in particular of diseases selected from the group of proliferative diseases, immune diseases, in particular autoimmune diseases, infectious diseases, vascular diseases, rheumatoid diseases, inflammatory diseases and other diseases associated with an increase or decrease of the expression of EGFR. The detection of the marker *in vitro* in, for example, a biopsy of a patient or *in vivo* in a patient by, for example, tomographic methodologies will allow the detection of sites of neovascularization within a specimen or within a patient.

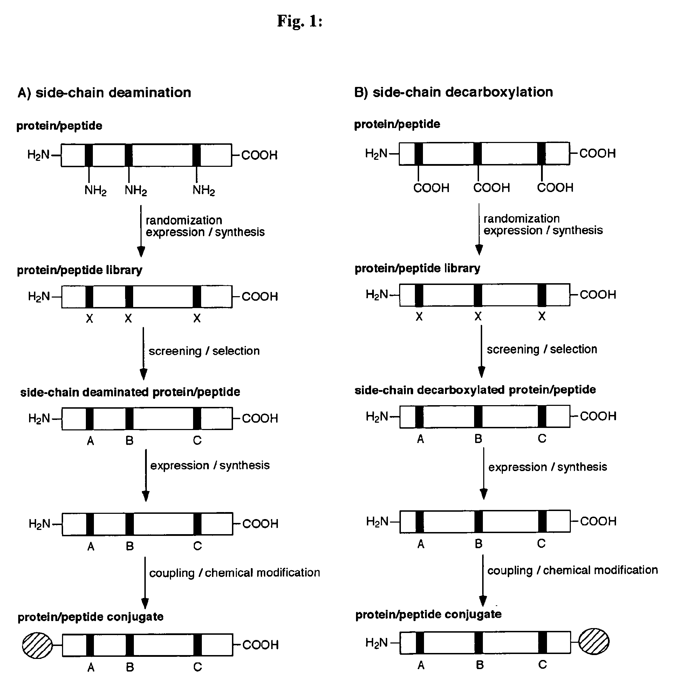

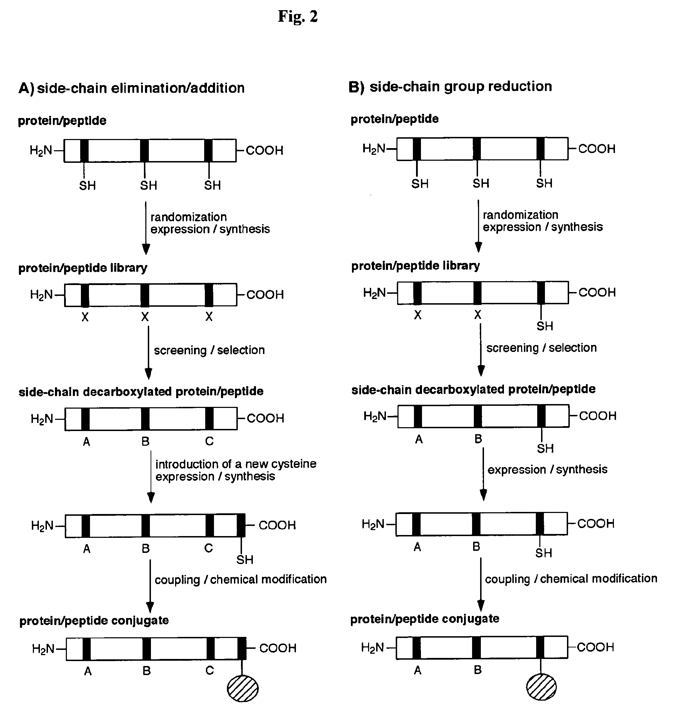

The novel approach , which led to the generation of EGF devoid of Lys residues capable of a site-specific coupling to other compounds without the prior art disadvantages and which still showed the desired binding activity can equally employed to other peptides and proteins. In one approach peptides or polypeptides which are devoid of internal side-chain amino- or carboxyl groups are generated, which allows for a site-specific coupling to either the N-terminal amino group or the C-terminal carboxyl group (Fig. 1). For the generation of peptides or polypeptides which can be coupled through the N-terminus, functional molecules are selected from a library containing randomized amino acids at the lysine positions (Fig. 1A). The essential feature of these libraries is that these randomized positions do not contain lysine residues. Thus, selection is driven towards active molecules which do not contain side-chain amino groups but possess other acceptable residues at these positions. This approach can also be applied to carboxyl group-containing amino acids (aspartic acid and glutamic acid) randomizing these positions with a selection of amino acids devoid of aspartic and glutamic acids (Fig. 1B). Further applications of this approach include the generation of peptides or polypeptides containing a unique internal or terminal amino acid with a side-chain group permissive to coupling, for example, the sulfhydryl group of cysteine, the guanidino group of arginine, the phenolate ring of tyrosine, the indol ring of tryptophan, the thioether of methionine, the imidazole ring of histidine, or a hydroxyl-containing amino acid such as group of serine or threonine. Threonine which might be also part of glycosylation sites which allows for the coupling through the carbohydrate moiety (Fig. 2). This is either achieved by randomization of all of the selected residues and introducing an additional reactive residue at selected positions, e.g. the N- or C-terminus (Fig. 2C), or by randomization of all but one of the selected residues (Fig. 2D). Randomization is either introduced at the genetic level (preferable in case of proteins and peptides, see table 1 for examples of useful codons) or synthetically (in case of peptides or short polypeptides).

Thus, a further aspect of the present invention is a method for producing a modified binding polypeptide, which is suitable for site-directed coupling, (see Fig. 1 and 2.) comprising the step of:
modifying a polynucleotide encoding the binding polypeptide, which is to be modified, by identifying within the reading frame of the polynucleotide all codons with the sequence:
   a) AAA and AAG encoding Lys and replacing this (these) codon(s) with (a) codon(s) NNN excluding AAA and AAG;
   b) AAA and AAG encoding Lys and replacing this (these) codon(s) with (a) codon(s) NNN excluding AAA and AAG and all codons with the sequence CGT, CGC, CGA, CGG, AGA, and AGG encoding Arg and replacing this (these) codon(s) with (a) codon(s) NNN excluding CGT, CGC, CGA, CGG, AGA, and AGG;
   c) GAT and GAC encoding Asp and replacing this (these) codon(s) with (a) codon(s) NNN excluding GAT and GAC and all codons with the sequence GAA and GAG encoding Glu and replacing this (these) codon(s) with (a) codon(s) NNN excluding GAA and GAG;
   d) TGT and TGC encoding Cys and replacing all but one of this (these) codon(s) with (a) codon(s) NNN excluding TGT and TGC;
   e) TCT, TCC, TCA, TCG, AGT and AGC encoding Ser and replacing all but one of this (these) codon(s) with (a) codon(s) NNN excluding TCT, TCC, TCA, TCG, AGT and AGC and all codons with the sequence ACT, ACC, ACA and ACG encoding Thr and replacing all but one of this (these) codon(s) with (a) codon(s) NNN excluding ACT, ACC, ACA and ACG;
   f) ATG encoding Met and replacing all but one of this (these) codon(s) with (a) codon(s) NNN excluding ATG;
   g) TAT and TAC encoding Tyr and replacing all but one of this (these) codon(s) with (a) codon(s) NNN excluding TAT and TAC;
   h) TGG encoding Trp and replacing all but one of this (these) codon(s) with (a) codon(s) NNN excluding TGG; and/or
   i) CAT and CAC encoding His and replacing all but one of this (these) codon(s) with (a) codon(s) NNN excluding CAT and CAC;
wherein N has the meaning: A, C, G or T. In case that the nucleic acid to be modified is a RNA rather than a DNA sequences the nucleotide T in above listing a) to i) is always replaced by the nucleotide U, e.g. for f) the codon would be AUG and it would be replaced with NNN excluding AUG, wherein N has the meaning: A, C, G, or U.

The term "reading frame" designates which nucleotide triplets of a polynucleotide encoding a protein are taken together to form a "codon", i.e. a coding nucleotide triplet, which is recognized by a t-RNA carrying a specific amino acid. A series of adjacent codons determines the amino acid sequence of the encoded polypeptide. A double stranded DNA has six potential reading three on each strand, while a single stranded DNA or RNA sequence, as may present in a DNA or RNA virus or a messenger RNA has three potential reading frames. Viruses often encode more than one protein with one stretch of nucleic acid by utilizing alternative reading frames, however, at least for higher eukaryotes usually only one reading frame within a given nucleic acid sequence encodes a protein The first nucleotide triplet is in most cases ATG encoding Met, which is preceded by initiation sites for protein synthesis recognized by the ribosome. The mechanisms of initiation of protein translation have been extensively studied in the past and have been described, for example, in Clark B.F. and Petersen H.F. (1984) Gene Expression. The translational step and its control, Munksgaard, Copenhagen. If there are doubts about which of the possible reading frame is used utilized to encode a particular protein it is also possible to partially sequence the encoded protein by routine techniques including micro-Edmann degradation or masspectrometry. The protein sequence will in turn allow the determination of the reading frame used. Thus, the averaged skilled practitioner is able to determine the reading frame of a given protein to be modified and identify the codons which needs to be replaced.

The term "binding polypeptide" as used through out the specification refers to polypeptides, which are capable to specifically interact with other chemical moieties, in particular with extracellular or intracellular structures of cells, in particular mammalian cells, or structures surrounding cells like connective tissue. Examples of extracellular structures are receptors including but not limited to VEGFR, EGFR, ErbB2, ErbB3, ErbB4, PDGFR, TGFα-R, TGFβ-R, KGFR, SDGFR, FGFR, IGF-1R, HGFR, , neurotrophine receptors TrkA, TrkB, TrkC, and LNGFR, BMF-R, bombesin receptor, M-CSFR, GM-CSF-R, thrombopoietin receptor, erythropoietin receptor, c-kit, SDGF-R, oncostatin receptor, PDEGF-R, endothelin receptor; cytokine receptors, in particular IL-1R, IL-2R, IL-3R, IL-4R, IL-5R, IL-6R, IL-7R, IL-8R, IL-9R, IL-10R, IL-12R, IL-13R, IL-14R, IL-15R, interferon α, β or γ-receptor, tumor necrosis factor receptors such as TNFα-R, TNFβ-R; chemokine receptors, in particular RANTES receptor, MCAF receptor, MIP-1α or β receptor, NAP receptor, β-thromboglobulin receptor; peptide hormone receptors such as SRH receptor, SIH receptor, STH receptor, MRH receptor, MSH receptor, PRH receptor, PIH receptor, prolactin receptor, LH-RH receptor, FSH-RH receptor, LH/ICSH receptor, FSH receptor, TRH receptor, TSH receptor, CRH receptor, ACTH receptor, angiotensin receptor, kinine receptor,; adhesion molecule receptors, in particular integrins α₁β₁, α₂β₁, α₃β₁, α₄β₇, α₅β₁, α₅β₃, α_{IIb}β₃, α_{M}β₂, α₆β₁, α₆β₄, α₇β₁, α₈β₁, α_{X}β₂, αᵥβ₁, αᵥβ₃, αᵥβ₅, αᵥβ₆, αᵥβ₇, αᵥβ₈, LFA-1-R, MAC-1-R, VLA-4R, PECAM receptor, vitronectin receptor, GMP-140 receptor, ICAM-1 receptor, VCAM-1 receptor, fibronectin receptor, laminin receptor, B7 receptor, CD28 receptor, CD40 receptor, CD40L receptor and selectin receptors; viral coat proteins; and bacterial surface proteins; and lipid or polysaccharide components accessible on the surface of the cell. Examples of intracellular structure are structural proteins, e.g. actin, keratin, vimetin and laminin, motor proteins, e.g. α/β-tubulin, dynein and kinesin, transcription factors, e.g. hormone receptors and structures surrounding cells include, for example, collagen, vitronection, laminin or fibronection.

The binding polypeptides can be naturally or non-naturally occurring. In the case of naturally occurring binding polypeptides they are polypeptides, which are naturally found in an animal and interact with extracellular or intracellular structures of cells, in particular mammalian cells, or surrounding cells like connective tissue. Examples of such binding polypeptides are the ligands for above indicated extracellular or intracellular structures or structures surrounding a cell. Naturally occurring binding polypeptides, which can be modified according to the method of the present invention also include antibodies, or fragments thereof, like Fv fragments Fab- or F(ab)₂-fragments. Non-naturally occurring binding polypeptides include polypeptides that have been selected to bind to a target chemical moiety like, for example, a receptor and engineered versions of naturally occurring binding peptides like, for example, single chain antibodies or ligand dimmers or multimers.

The selection of which of the alternative method steps a) to i) are employed for a given method of the invention depends on the respective binding polypeptide, in particular on the amino acid composition and the position of the binding domain within the binding polypeptide. In most cases it will be desirable to select those codons for modification according to steps a) to i), which are least abundant, i.e. which require the least modification of the polynucleotide to arrive at a polynucleotide which has none (in cases a) to c)) or only one codon (in cases (d) to i)) encoding the respective amino acid left. The position of the binding domain within the binding polypeptide is important, since in most cases the amino acid used for site-specific coupling will be selected to be outside the binding region.

In particular in those embodiments of the method of the present invention in which all but one of the codons encoding Cys, Ser, Thr, Met, Tyr, Trp or His are modified, i.e. d) to i), the one codon of the respective type of amino acid which is not modified will preferentially be present in the vicinity of the beginning or end of the coding region, since the resulting polypeptide is in most cases preferentially coupled via a amino acid residue in the vicinity or at the N- or C-terminus. For this purpose it is also possible to engineer a polynucleotide encoding a binding protein with an additional Cys, Ser, Thr, Met, Tyr, Trp or His at the N- or C-terminus. However, in some cases it might be desirable to carry out the site-specific coupling between an internal amino acid of the binding polypeptide and another chemical moiety. An example of such a case would be a protein in which the N- and the C-terminus form the binding region that specifically interacts with the binding partner of the binding protein. In this case a coupling via the N- or C-terminus would be detrimental to the binding of the binding polypeptide to the binding partner. Therefore, it is also possible to select the one codon which is not replaced within the coding region. It is also envisioned that a polynucleotide encoding a binding polypeptide to be modified is initially modified to include an additional codon encoding Cys, Ser, Thr, Met, Tyr, Trp or His at a position of choice and that in a second step the method of the present is employed to alter all other Cys, Ser, Thr, Met, Tyr, Trp and/or His residues. In most cases only one type amino acid out of Cys, Ser, Thr, Met, Tyr, Trp or His will be modified in a given binding polypeptide to create only one residue capable of site-specific coupling, however, if the site-specific coupling is carried out on a Ser or Thr residue all but one Ser or Thr residue have to be removed from the polypeptide, since most coupling reagents will not differentiate between Ser and Thr. Lys and Arg usually both react coupling reagents specific to free -NH₂ groups, however, usually the reaction proceeds more readily with Lys than with Arg. Therefore, if the modified binding polypeptide will later coupled via its N-terminus at least all Lys residues have to be removed from the polypeptide and preferably all Lys and most or all Arg residues. Similarly, Glu and Asp react with coupling agents specific to free -COOH groups and, therefore, if C-terminal coupling is to be carried out it is necessary to remove all Asp and Glu residues from the binding polypeptide.

In some case it might be desirable to carry out two or more site-specific coupling reactions with the binding polypeptide and in those cases any combinations of the alternative method steps a) to i) can be carried out. For example it is possible to remove all Lys residues from the binding polypeptide to allow site-specific coupling via the N-terminus of the polypeptide and in addition remove all Cys residues with the exception of one at the C-terminus, which will then allow C-terminal coupling of the binding polypeptide.

Thus, based on the binding properties of the binding polypeptide to be modified, i.e. N-terminal, C-terminal or internal binding domain, and the amino acid composition and the number of site-specific coupling reactions, which are to be carried out the averaged skilled practitioner is able to select the method step a) to i) or a combination of two or more of those most suitable for the respective binding polypeptide without undue experimentation.

A wide variety of coupling agents, which are specific to free -NH₂ or -COOH groups are known in the art as well as coupling groups, which specifically or preferentially react with Cys, Ser, Thr, Met, Tyr, Trp or His.

Coupling to single reactive groups can be achieved for amino acids containing: free amino groups, e.g. N-terminal amino acid residue, Lys or Arg, with, for example, isocanates, isothicyanates, acyl azides, NHS (N-hydroxysuccinimide) esters, sulfonyl chlorides, aldehydes and gloxals, epoxides and oxiranes, carbonates, arylating agents, imidoesters, carbodiimides, and anhydrides; sulfhydryl groups, e.g. Cys, with, for example, haloacetyl and alkyl halide derivatives, maleimides, aziridines, acryloyl derivatives, arylating agents, and thiol-disulfide exchange reactions using pyridyl disulfides, TNB-thiol, or disulfide reductants; carboxyl groups, e.g. Asp or Glu, with, for example, diazoalkanes and diazoacetyl compounds, carbonyldiimidazole, and carbodiimides; hydroxyl groups, e.g. Ser or Thr, with, for example, epoxides and oxiranes, carbonyldiimidazole, N,N'-disuccinimidyl carbonate or N-hydroxysuccinimidyl chloroformate, periodates, alkyl halogens, and isocyanates; thioester groups with, for example, haloacetyl and alkyl halide derivates; aldehyde- and ketone groups generated from carbohydrates reactive with, for example, hydrazine derivatives or by forming Schiff bases; reactive hydrogen groups, e.g. Tyr or His with, for example, diazonium derivatives and oxidizing agents. (An extensive overview of coupling procedures can be found in: Hermanson, G.T. (1996) Bioconjugate techniques, Academic Press).

The length of the binding polypeptide, which is to be modified is not limited, however, given that the longer the binding polypeptide the more amino acids of the type of amino acid , which has been chosen to be removed, will have to be removed. Therefore, in a preferred embodiment the binding polypeptide, which is to be modified is smaller than 300 amino acids, preferably smaller than 200 amino acids, and more preferably smaller than 100 amino acids.

A large variety of methods are known in the prior art to introduce site specific mutations into polynucleotide sequences, which can all without limitations be employed to replace the selected codons within the polynucleotide. A preferred method uses DNA primers, which are degenerated at the codon positions to be modified, in PCR reactions to amplify a part or all of the sequence of the binding polypeptide. In cases that only a part of the binding polypeptide is amplified in such a PCR reaction this part can be combined with one or more additional parts of polynucleotides encoding the binding protein to form a polynucleotide encoding the entire binding protein. The methods which can be used are entirely routine and are contained in Standard Laboratory Manuals like, for example, Sambrook et al. (2001) Molecular Cloning: a Laboratory Manual, 3^{rd} Edition Cold Spring Harbor Laboratory Press.

In a preferred embodiment of the method for producing a modified binding polypeptide, which is suitable for site-directed coupling, the method comprises the step of:
modifying a polynucleotide encoding the binding polypeptide, which is to be modified, by identifying within the reading frame of the polynucleotide all codons with the sequence:
   j) AAA and AAG encoding Lys are replaced with a sequence selected from the group consisting of BNK, NNT, NBK, NBK, KNK, NHT, BHK, DNT, VVT, HHT, VRT, HMT, TDK, BWT, TKK, TWC, KMT, AVT, and TWC;
   k) AAA and AAG encoding Lys and CGT, CGC, CGA, CGG, AGA, and AGG encoding Arg are replaced with a sequence selected from the group consisting of NHT, KNK, BHK, DNT, HHT, NWT, HMT, TDK, BWT, TKK, KMT, and TWC;
   l) AAA and AAG encoding Lys are replaced with a sequence selected from the group consisting of BNK, NNT, NBK, NBK, KNK, NHT, BHK, DNT, VVT, HHT, VRT, HMT, TDK, BWT, TKK, TWC, KMT, AVT, and TWC and all codons with the sequence CGT, CGC, CGA, CGG, AGA, and AGG encoding Arg are replaced with a sequence selected from the group consisting of NHK, NHT, KNK, BHK, DNT, HHT, NWT, HMT, BWT, TDK, TKK, RAK, and TWC;
   m) GAT and GAC encoding Asp and GAA and GAG encoding Glu are replaced with a sequence selected from the group consisting of HNK, NBK, MNK, HHT, MRK, TKK, and TWC, and;
   n) TGT and TGC encoding Cys are replaced all but one of with a sequence selected from the group consisting of VNK, NHK, NNG, VVK, BHK, MNK, VVT, NWT, RRK, VRT, MRK, BWT, NKT, RAK, RRG, KMT, AVT, and RAG,;
   o) TCT, TCC, TCA, TCG, AGT and AGC encoding Ser and ACT, ACC, ACA and ACG encoding Thr are replaced all but one of with a sequence selected from the group consisting of RAK, TDK, TKK, VWT, BWT, NWT, RRG, and TWC;
   p) ATG encoding Met are replaced all but one of with a sequence selected from the group consisting of NVK, BNK, NNT, VVK, NHT, KNK, BHK, DNT, VVT, HHT, NWT, RRK, VRT, HMT, MRK, TDK, BWT, TKK, RAK, RRG, TWC, and RAG;
   q) TAT and TAC encoding Tyr are replaced all but one of with a sequence selected from the group consisting of VNK, NNG, VVK, MNK, VVT, RRK, MRK, VRT, TKK, RAK, RRG, AVT, and RAG;
   r) TGG encoding Trp are replaced all but one of with a sequence selected from the group consisting of VNK, NHK, NNT, VVK, BHK, MNK, VVT, NWT, RRK, VRT, MRK, BWT, RAK, RRG, AVT, TWC, and RAG; and/or
   s) CAT and CAC encoding His are replaced all but one of with a sequence selected from the group consisting of NNG, NBK, NBK, RRK, TDK, TKK, RAK, RRG, KMT, AVT, TWC, and RAG;
wherein R has the meaning: A or G; K has the meaning: G or T; M has the meaning: A or C; W has the meaning: A or T; B has the meaning: C, G or T; D has the meaning: A, G or T; H has the meaning: A, C or T; V has the meaning: A, G or C; N has the meaning: A, C, G or G. Again in cases in which the nucleic acid to be modified is an RNA T in above listing has in all instances the meaning U. The general principles outlined for the selection of the modification steps a) to i) outlined above similarly apply to the modification steps j) to s).

In a preferred embodiment the method of the present invention, further comprises the step of coupling the modified polynucleotide to at least one additional polynucleotide encoding a polypeptide to produce a fusion polynucleotide encoding a modified fusion polypeptide. Thus, after modification of the polynucleotide encoding the binding polypeptide it is possible to chemically or by enzymatic means, e.g. restriction digest and ligation, to add additional nucleotides encoding further proteins. Such a modification will often be required, if the modified polypeptide is to be tested for its capacity to bind to the binding partner of the unmodified polypeptide. Such assays include, for example, phage display, where the modified binding polypeptide will be fused to a phage envelope protein. Thus, the modified polynucleotide encoding the modified binding polypeptide can be introduced into any polynucleotide sequence, in particular into plasmids and vectors as outlined above with respect to modified EGF.

The method of the present invention comprises in a preferred embodiment the additional step of expressing the modified polynucleotide or the fusion polynucleotide to produce a modified binding polypeptide or modified binding fusion polypeptide. This expression can be carried out using any of the vector system and regulatory elements known in the art and examples of which were indicated above with respect to the expression of modified EGF.

The method of the present invention comprises in a preferred embodiment the additional steps of:
a) incubating the modified binding polypeptide or fusion polypeptide or viral particles or cells displaying the modified binding polypeptide or fusion polypeptide with at least one binding partner of the binding polypeptide, and
b) selecting the modified binding polypeptide or fusion polypeptides or viral particles or cells displaying the modified binding polypeptide or fusion polypeptide, which shows at least 10% of the binding strength of the binding polypeptide to the binding partner.

The term "binding partner" refers to the chemical moiety, which specifically binds to the unmodified binding polypeptide. Examples of binding partners were given above and include extracellular and intracellular structures as well as structures surrounding cells like, for example, connective tissue. A modified binding polypeptide, which is selected according to the method of the present invention is a polypeptide that has at least 10% (e.g., at least: 10%, 20%; 30%; 40%; 50%; 60%; 70%; 80%; 90%; 95%; 98%; 99%; 99.5%; or 100% or even more) of the ability of the unmodified binding polypeptide to bind to its binding partner. It is preferred that the modified binding polypeptide has at the same binding activity as the unmodified polypeptide. Binding assays for assessing the binding the modified polypeptide to their respective binding partner are well known in the art and include isothermal titration calorimetry (Lemmon et al. (1997) EMBO J. 16, 281-294), titration or competition experiments with radiolabeled binding polypeptide (Campion et al. (1993) J. Biol. Chem. 268, 1742-1748), and surface plasmon resonance measurements (Lenferink et al. (2000) J. Biol. Chem. 275, 26748-26753) and further methods are also described herein below.

The assessment of the binding strength can be carried out between one by one between a series of modified binding polypeptides and the binding partner or in parallel. For the later approach high throughput interaction screens can be used, which are all well established in the prior art and include immobilization of different modified polypeptides on spots on the surface of, for example, chips or membranes. Alternatively, display technologies such as phage display, bacterial display, yeast display, viral display, mammalian cell display, in vitro display technologies such as ribosome display and mRNA display, or in vitro compartimentalization systems can be applied for the selection of modified polypeptides (Amstutz et al. (2001) Curr. Op. Biotechnol. 12, 400-405). The key feature of these technologies is the physical linkage of genetic information and the expressed protein in large combinatorial libraries, which allow selection of polypeptides through binding or activity, amplification of the material after selection in biological or synthetic systems and thus enrichment of desired polypeptides and the genetic information encoding them..

In a preferred embodiment the method of the present invention comprises the further step of site-specific coupling of the modified binding polypeptide or fusion polypeptide to at least one chemical moiety. The site specific coupling can be carried out by using coupling reagents known in the art and which have been extensively reviewed in: Hermanson, G.T. (1996) Bioconjugate techniques, Academic Press. Some exemplary reagents, which allow site specific coupling have already been given above In a preferred embodiment the chemical moiety is coupled to:
aa) a N-terminal amino group of the modified binding polypeptide or fusion polypeptide and wherein the modified binding polypeptide has been modified according to alternative a) or b) or according to alternative j) to l);
bb) a C-terminal carboxyl group of the modified binding polypeptide or fusion polypeptide and wherein the modified binding polypeptide has been modified according to alternative c) or according to alternative m);
cc) the single Cys residue of the modified binding polypeptide or fusion polypeptide and wherein the modified binding polypeptide has been modified according to alternative d) or according to alternative n);
dd) the single Ser residue of the modified binding polypeptide or fusion polypeptide and wherein the modified binding polypeptide has been modified according to alternative e) or according to alternative o);
ee) the single Met residue of the modified binding polypeptide or fusion polypeptide and wherein the modified binding polypeptide has been modified according to alternative f) or according to alternative p);
ff) the single Tyr residue of the modified binding polypeptide or fusion polypeptide and wherein the modified binding polypeptide has been modified according to alternative g) or according to alternative q);
gg) the single Trp residue of the modified binding polypeptide or fusion polypeptide and wherein the modified binding polypeptide has been modified according to alternative h) or according to alternative r); and/or
hh) the single His residue of the modified binding polypeptide or fusion polypeptide and wherein the modified binding polypeptide has been modified according to alternative i) or according to alternative s).

As described with respect to the general concept of selection of the codon(s) to replace the modified binding polypeptide, the modified polypeptide can be site-specifically coupled to one, two or more chemical moieties, depending on whether the binding polypeptide comprises only one, two or more amino acid residues, which can be site-specifically coupled like, for example, only one amino acid with a free amino group and only one amino acid with a free carboxy group.

The term "chemical moiety" has in this context the same meaning as outlined above with respect to the coupling of EGF to a chemical moiety. In a preferred embodiment the chemical moiety is selected from the group consisting of a spacer, a marker, a tag, a lipid, in particular a phospholipid, a drug, a capping group, a polypeptide and a spacer attached to a second chemical moiety. In an even more preferred embodiment the polypeptide is selected from the group consisting of a cytokine, a chemokine, a growth factor, an adhesion molecule, an antibody light and/or heavy chain, a single chain antibody, a toxin, an enzyme, a receptor ligand, a lytic peptide, a membrane insertion sequence and a fluorescent protein or fragments thereof. The other chemical moieties, which can be coupled to the modified binding polypeptides or modified binding fusion polypeptides, which are produced according to the method of the present invention, i.e. a spacer, a marker, a tag, a lipid, in particular a phospholipid, a drug, a capping group, and a spacer attached to a second chemical moiety have the same meaning and preferred meanings as outlined above with respect to EGF.

Furthermore, the modified binding polypeptides produced as outlined above can in a further step be incorporated, coupled or attached to any of the structures and preferred structures described above with respect to EGF like, for example, liposomes, virosomes, microsphere, niosomes, dendrimeres, stabilizers, buffers, excipient, additives. In addition they can be mixed or formulated in compositions as therapeutics or diagnostics as outlined above.

The term "binding polypeptide" as stated above encompasses all polypeptides, showing specific binding to another chemical moiety, however, in a particular preferred embodiment of the method of the present invention the binding polypeptide is selected from the group consisting of growth factors, in particular VEGF, EGF, Her2/neu, PDGF, TGFα, TGFβ, KGF, SDGF, FGF, IGF, HGF, NGF, BDNF, neurotrophine, BMF, bombesin, M-CSF, GM-CSF, thrombopoietin, erythropoietin, SCF, SDGF, oncostatin, PDEGF, endothelin; cytokines, in particular IL-1, IL-2, IL-3, IL-4, IL-5, IL-6, IL-7, IL-8, IL-9, IL-10, IL-12, IL-13, IL-14, IL-15, interferon α, β or γ, tumor necrosis factors such as TNFα, TNFβ; chemokines, in particular RANTES, MCAF, MIP-1α or β, NAP, β-thromboglobulin; peptide hormones such as SRH, SIH, STH, MRH, MSH, PRH, PIH, prolactin, LH-RH, FSH-RH, LH/ICSH, FSH, TRH, TSH, CRH, ACTH, agiotensin, kinine, histamine; adhesion molecules, in particular LFA-1, MAC-1, VLA-4, PECAM, vitronectin, GMP-140, ICAM-1, VCAM-1, fibronectin, laminin, B7, CD28, CD40, CD40L and selectins; viral coat proteins; and bacterial surface proteins.

The method of the present invention leads to the identification of modified binding polypeptides, which are capable of site-specific coupling to chemical moieties without substantially impairing the binding strength of the binding polypeptide due to unwanted crosslinking. Therefore, the modified polypeptides produced according to the method of the present invention can be used to target any chemical moiety coupled to the polypeptide or can be used as such to specifically localize within the body in regions that comprise the binding partner. Therefore, another aspect of the invention is the use of a modified binding polypeptide or fusion polypeptide producable by the method of the invention for the manufacture of a medicament or diagnostic for the prevention, treatment or diagnosis of a disease, which is characterized by an increased or decreased amount of at least one binding partner of the binding polypeptide in diseased tissue or cells involved in the disease. In a preferred embodiment the disease is selected from the group of diseases consisting of proliferative diseases, immune diseases, infectious diseases, vascular diseases and rheumatoid diseases.

The following examples are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims. All references cited are incorporated herein by reference.

### Brief Description of the Figures and Drawings

**Fig. 1:** Schematic representation of the strategy to isolate side-chain deaminated ligands (A) or side-chain decarboxylated ligands (B). Randomization in approach A is performed by using sets of amino acids (X) devoid of lysines or lysines and arginines. Randomization in approach B is performed by using sets of amino acids (X) devoid of aspartic acids (Asp) and glutamic acids (Glu).
**Fig. 2**: Schematic representation of the strategy of side-chain elimination/addition (**A**) or side-chain group reduction (**B**), both examplified for elimination or reduction of cysteine residues. In these examples randomization is performed by using sets of amino acids (X) devoid of cysteines (Cys). Both approaches can be applied equally for any other amino acid residues.

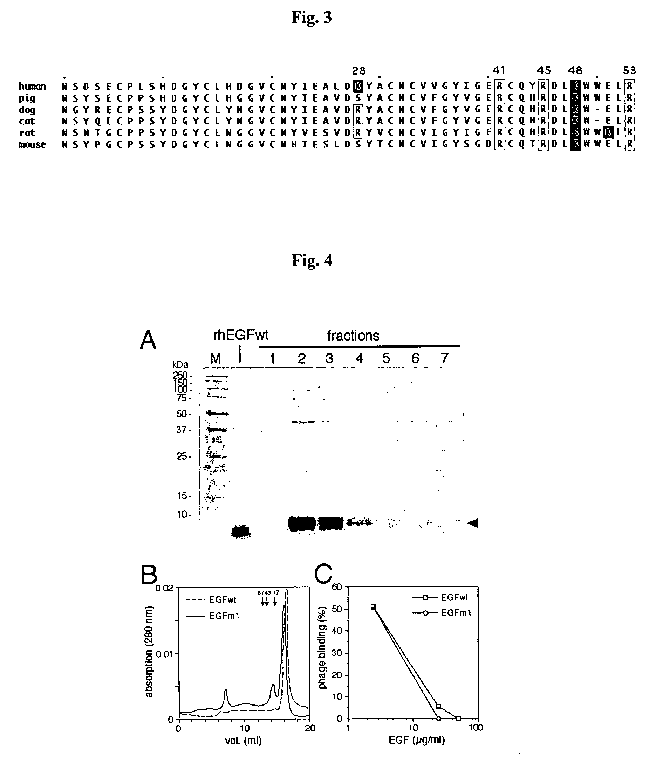

**Fig. 3**: Amino acid sequences of EGF of various species. The positions of lysine and arginine residues is highlighted by grey boxes.
**Fig. 4:** (**A**) IMAC purification of recombinant HisEGFm1 from bacterial periplasm. Eluted fractions were analyzed on a 20% SDS-Tricine gel (HisEGFm1 is indicated by an arrowhead). Recombinant human EGF was included as control. (**B**) Size exclusion chromatography of purified EGFwt and HisEGFm1 on a superose 12 column. The positions of BSA (67 kDa), Ovalbumin (43 kDa) and Myoglobin (17 kDa) are indicated by arrows. (**C**) Competition of binding of fdEGF to A431 by EGFwt or HisEGFm1.

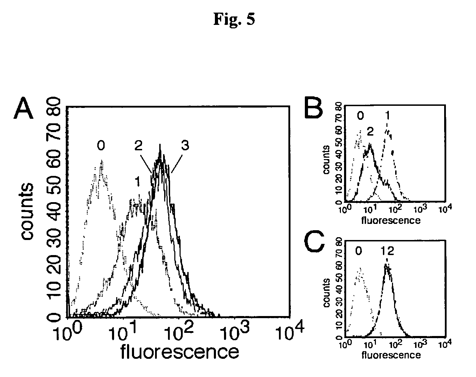

**Fig. 5:** Binding of FITC-labeled EGFm1 to A431. A) FITC-labeled HisEGFm1 was incubated with A431 at varying concentrations and binding was analyzed by flow cytometry (0, cells without EGFm1; 1, 17 ng/ml; 2 50 ng/ml; 3, 170 ng/ml). B) Competition of FITC-labeled EGFm1 at 170 ng/ml without (1) or with (2) excess amounts of unlabelled EGFwt. C) Control experiments with BSA.

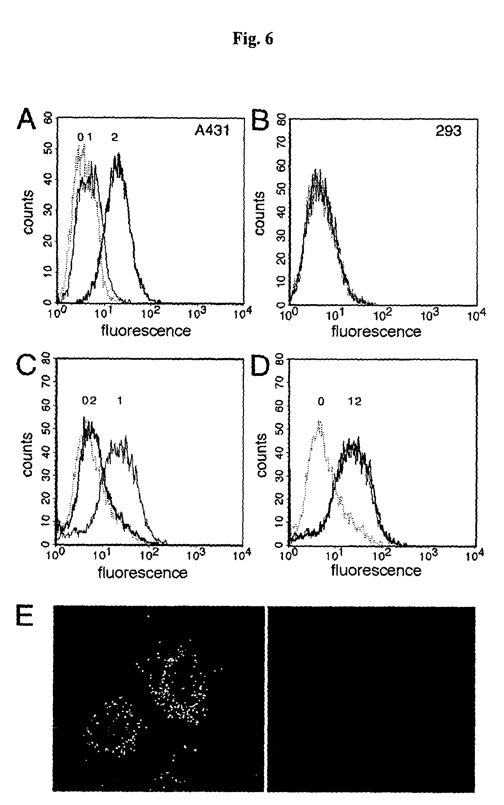

**Fig. 6: (A)** Binding of EGFm1 liposomes to A431 cells. EGFm1 liposomes (2) showed specific binding to A431 cells, in contrast to unconjugated liposomes (1), which did not show only marginal binding to A431 cells (0). No binding was observed with 293 cells (**B**). Excess amounts of EGFwt completely inhibited binding of EGFm1 liposomes to A431 cells (**C**), in contrast to BSA (**D**) which did not show any effects (0 = cells alone, 1 = EGFm1 liposomes, 2 = EGFm1 liposomes + EGFwt or BSA. (**E**) Internalization study incubation A43 cells with EGFm1 liposomes for 6 hrs at 37°C (left, fluorescence microscopy image; right, phase-contrast image). Internalization is evidenced by a perinuclear accumulation of fluorescence.

### Examples

### Example 1: Generation of lysine-deficient EGF libraries

For the selection of lysine-deficient EGF variants we generated two EGF libraries. The first EGF library (EGF1) was randomized at positions K28 and K48 using the triplet BNK coding for 15 amino acids (LVFYWRHDECQSGAP see SEQ ID. No 31). The second library (EGF2) was randomized at positions K28, R41, R45, K48, and R53 using the triplet BNK for K28 and the triplet VVT coding for 9 mainly hydrophilic amino acids (RHTSDNGAP see SEQ ID. No 32) for the remaining positions (see Fig. 3 for EGF sequence). Mutagenesis of arginine residues was included since the guanidinium group can serve as target for amino-reactive coupling reagents under certain conditions. Library EGF1 encodes a diversity of 225 possible permutations and library EGF2 of 9.8 x 10⁴ permutations. Both libraries are devoid of lysine residues but allow arginine at the randomized positions.

EGF libraries were generated by a two fragment ligation procedure. Fragment 1 was produced by PCR with primers EGFSfiback (5'-TAT GCG GCC CAG CCG GCC ATG GCC AAT AGT GAC TCT GAA TGT-3' see SEQ ID. No 33) and EGFHindFor (5'-GTC CAA AGC TTC AAT ATA CAT GCA-3' indicated in SEQ ID. No 34) introducing a *Hind* III site between codons 24-26. Fragment 2 was produced by PCR with primers K1Back (5'-ATT GAA GCT TTG GAC BNK TAT GCA TGC AAC TGT GGT-3' see SEQ ID. No 35) and either K2For (5'-CAG TGC GGC GCG ACG CAG TTC CCA CCA MNV CAG GTC TCG GTA CTG ACA-3 see SEQ ID. No 36) or K3For (5'-CAG TGC GGC CGC ABB CAG TTC CCA CCA ABB CAG GTC ABB GTA CTG ACA ABB CTC CCC GAT GTA GCC AAC-3' see SEQ ID. No 37), thus introducing randomized codons and a *Hind* III site. Fragment 1 was digested with *Sfi* I and *Hind* III and fragment 2 with *Hind* III and *Not* I. The two fragments were then cloned into phagemid vector pHEN3 digested with *Sfi* I and *Not* I. Library sizes (positive inserts) were 4 x 10⁵ for EGF and 4.6 x 10⁴ for EGF2.

### Example 2: Selection of lysine-deficient EGF variants

Selections were performed on EGF-expressing A431 cells. A431 (5 x 10⁶ cells) were resuspended in ice-cold DMEM containing 10% FCS, phage were added and incubated for 1 hr on ice. Cells were then washed 6 times with ice-cold medium and once with PBS. Cell pellet was resuspended in 500 µl 100 mM triethylamine and incubated for 7 min. Cells were pelleted and supernatant was neutralized by adding 250 µM 1 M Tris-HCl pH 7.4. 400 µl of the phage solution was added to 10 ml log-phase TG1 and incubated for 60 min at 37°C. Phage were titrated by plating dilutions onto 2xTY, amp, 1% glucose plates. Phage eluted from the first round were directly used for the second round without any amplification.

After two rounds between 92 to 100% of the eluted phage stained positive in immunofluorescence analysis of binding to A431. The sequence data of several positive clones is summarized in table 2. A preference for hydrophobic residues was observed for phage selected from library EGF1. Only one clone (# 3) contained an arginine at position 48 instead of lysine (table 2). Most interestingly, all phage selected from the EGF2 library had the original arginine at position 41 indicating that R41 is essential for binding of EGF to its receptor. Only one clone (# 9) contained the original arginine at position 45 (table 2). In summary, from both libraries we selected several EGF variants devoid of lysine residues. Furthermore, the number of arginine residues was reduced from 3 to 1 in most of the EGF variants selected from library EGF2.

In summary, applying EGF libraries we were able to select EGF variants with full binding activity which are deficient of lysine residues and which lack two of the three arginine residues. A diverse set of residues was found at positions 28, 45, 48, and 53. We did not observe a bias towards a conservative substitution of the lysine residues by arginine residues. This is in accordance with previous findings, which showed that the electrostatic property of K28 and K48 is not required for receptor-ligand association. Our results also confirmed the importance of arginine 41 for receptor binding of EGF established previously by site-directed mutagenesis and chemical modification experiments.

### Example 3: Recombinant expression of a lysine-deficient EGF variant

We selected EGFm1 (clone 6 of the EGF2 library selection) for soluble expression. EGFm1 containing a hexahistidyl tag at its N-terminus was purified by IMAC in soluble form from the periplasm of transformed BL21 DE3 cells. For this purpose, EGFm1 DNA was amplified with primers SfiHisEGF (5'-TAT GCG GCC CAG CCG GCC ATG GCC GGA CAT CAC CAT CAT CAC CAT GCG AAT AGT GAC TCT GAA TGT-3') and EGFstopm1 (5'-AGT CAG TGC GGC CGC TTA ACT CAG TTC CCA CCA ACT CAG-3') and cloned as *Nco* I / *Not* I fragment into pET22b (Novagen, Schwalbach, Germany). EGF containing a hexahistidyl-tag at the N-terminus was purified from the periplasm by immobilized metal affinity chromatography (IMAC) (Qiagen; Hilden, Germany) as described for recombinant scFv fragments (Kontermann et al., (1997) Immunotechnology 3, 137-144). EGF was further purified by FPLC size exclusion chromatography on a superose 12 column (Pharmacia, Freiburg, Germany). SDS-PAGE analysis was performed with 20% tricine gels.

Approximately 0.6 mg were purified from 1 litre of bacterial culture induced for 3 hours at 23°C. SDS-PAGE analysis confirmed the correct size of 7 kDa (Figure 4A). As expected EGFm1 migrated slightly slower than recombinant EGFwt (purchased from Promega (Mannheim, Germany) lacking the hexahistidyl tag. This was further confirmed by size exclusion chromatography (Figure 4B). This experiment also revealed the presence of molecules with a size of approximately 15 kDa in the EGFm1 preparation corresponding in size to dimeric EGF molecules.

Binding of purified EGFm1 to the EGF receptor was demonstrated by competition of binding of EGFwt-displaying phage to A431 cells by soluble EGFm1 or EGFwt (Figure 4C). A431 cells were incubated with 10¹¹ wild-type fdEGF phage and varying concentrations (2.5 - 50 µg/ml) of purified rhEGF or EGFm1 for 30 min. on ice. After washing cells with PBS bound phage were detected with mouse anti-M13 antibody (Pharmacia) and Cy3-labelled goat-anti-mouse antibody. Binding was analyzed by FACS and inhibition of phage binding by EGF was calculated from the mean fluorescence intensity. A titration of EGFm1 compared to EGFwt gave identical values with an IC₅₀ of approximately 0.5 µM indicating that EGFm1 binds with the same affinity as EGFwt to A431 cells.

### Example 4: Coupling of amino-reactive reagents to EGFm1

Reactivity of EGFm1 with amino-reactive reagents was demonstrated with FITC. EGFwt or EGFm1 (50 µg) were diluted to a conc. of 500 µg/ml and the pH was adjusted to 9.0 with carbonate buffer pH 9.5. FITC was added at a 10-fold molar excess and incubated overnight at 4°C. Labeled EGF was separated from FITC by gel filtration on sephadex 25 (Pharmacia, Freiburg, Germany). Fluorescence intensity was determined using a fluorescence spectrophotometer (Victor²; Wallac, Freiburg, Germany). The protein peak fractions of EGFwt had a fluorescence intensity of 84945 units compared to 29720 for EGFm1. Thus, the ratio of labeling of EGFwt to EGFm1 was 2.9, which is almost identical to the 3:1 ratio of amino groups present in these proteins. Fluorescein-labeled EGFm1 showed strong and concentration-dependent binding to A431 cells demonstrating that labeling did not interfere with binding to the EGF receptor (Figure 5A). In this experiment, binding of fluorescein-labeled EGFm1 to A431 reached saturation at approximately 50 ng/ml and could be competed with excess amounts of unlabeled EGFwt but not with BSA (Figure 5B, C).

### Example 5: Coupling of EGFm1 to liposomes

In a further experiment we coupled EGFm1 to pegylated liposomes. Lipids were purchased from Avanti (Alabaster, USA) and NHS-PEG₂₀₀₀-DSPE from Shearwater Polymers (now Nektar; Birmingham, USA). Liposomes consisting of neutral phospholipids, cholesterol, NHS-PEG₂₀₀₀-distearoylphosphatidylethanolamine (NHS-PEG₂₀₀₀-DSPE), and rhodamine-dipalmitoyl-phosphatidylethanolamin (Rh-DPPE) at a molar ratio of 6:3:1:0.03 were prepared from dried films by hydration with PBS pH 6.0. After extrusion through 50 nm filters, the pH was adjusted to pH 8 with NaOH and EGFm1 was immediately added at a conc. of 50 µg per 1 µmol lipid. After incubation for 16 hrs at room temperature unbound ligands were removed by gel filtration on sepharose 4B (Pharmacia, Freiburg, Germany). Thus, these liposomes display EGFm at the end of the PEG chain.

Incubation with A431 cells demonstrated a strong binding to these cells compared to the same liposomes lacking EGFm1 (Figure 6A). In contrast, no binding was observed with EGFR-negative 293 cells (Figure 6B). As observed before for FITC-labeled EGFm1, binding to A431 cells could be completely inhibited by excess amounts of EGFwt but not with BSA (Figure 6C, D). Incubation of A431 cells with EGFm1 liposomes at 37°C resulted in internalization as evidenced by a perinuclear accumulation of fluorescence (Figure 6E). No such pattern was observed incubating A431 cells at 4°C (not shown). In addition, no staining and internalization was seen with unconjugated liposomes or using 293 cells (not shown).

This finding demonstrates that EGF variants devoid of all lysine residues can be coupled to liposomal drug carrier systems without interfering with EGF binding activity resulting in target cell-specific liposomes.

**Table 1:**

| **Codons for biased amino acid usage and their encoded amino acids** | |
|---|---|
| Abbreviations of nucleotides: | |
| N = A,C,G,T | |
| V = G,A,C | |
| D = G,A,T | |
| B = G,T,C | |
| H = A,T,C | |
| W = A,T | |
| M=A,C | |
| R = A,G | |
| K = G,T | |
| Y=C,T | |
| S = G,C | |
| | |

| **A) Examples of triplets devoid of lysine-encoding codons** | |
|---|---|
| BNK | LVFYWRHDECQSGAP |
| NNT | LIVFYRHDCSTNGAP |
| NBK | LIMVFWRCSTGAP |
| KNK | LVFYWDESCGA |
| NHT | LIVFYHDSTNAP |
| BHK | LVFYHDESQAP |
| DNT | IVFYDSCTNGA |
| VVT | RHDSTNGAP |
| HHT | LIFYHSTNP |
| VRT | RHDSNG |
| HMT | YHSTNP |
| TDK | LFYWC |
| BWT | LIFYHD |
| TKK | LFWC |
| KMT | YDSA |
| AVT | TSN |
| TWC | FY |
| | |

| **B) Examples of triplets devoid of aspartic acid- and glutamic acid-encoding codons** | |
|---|---|
| NBK | |
| HNK | LIMFYWKRHCSTQNP |
| NBK | LIMVFWRSTGAP |
| MNK | LIMKRHSTQNP |
| HHT | LIFYHSTNP |
| MRK | KRHNQS |
| TKK | LFWC |
| TWC | FY |
| | |

| **C) Examples of triplets devoid of cyteine-encoding codons** | |
|---|---|
| NNG | |
| VNK | LIMVKRHDESTQNGAP |
| NHK | LIMVFYKHDESTQNAP |
| NNG | LMVWKRESTQGAP |
| VVK | KRHDESTQNGAP |
| BHK | LVFYHDESQAP |
| MNK | LIMKRHSTQNP |
| VVT | RHDSTNGAP |
| NWT | LIVFYHDN |
| RRK | KRDENSG |
| VRT | RHDSNG |
| MRK | KRHNQS |
| BWT | LIFYHD |
| NKT | LIMVF |
| RAK | KDEN |
| RRG | KREG |
| KMT | YDSA |
| AVT | TSN |
| RAG | KE |
| | |

| **D) Examples of triplets devoid of serine and threonine-encoding codons** | |
|---|---|
| RAK | KDEN |
| TDK | LFYWC |
| TKK | LFWC |
| BWT | LIFYHD |
| NWT | LIVFYHDN |
| RRG | KREG |
| TWC | FY |
| | |

| **E) Examples of triplets devoid of arginine-encoding codons** | |
|---|---|
| NHK | LIMVFYKHDESTQNAP |
| NHT | LIVFYHDSTNAP |
| KNK | LVFYWDESCGA |
| BHK | LVFYHDESQAP |
| DNT | IVFYDSCTNGA |
| HHT | LIFYHSTNP |
| NWT | LIVFYHDN |
| HMT | YHSTNP |
| BWT | LIFYHD |
| TDK | LFYWC |
| TKK | LFWC |
| RAK | KDEN |
| TWC | FY |
| | |

| **F) Examples of triplets devoid of methionine-encoding codons** | |
|---|---|
| NVK | WYKRHDECSTQNGAP |
| BNK | LVFYWRHDECQSGAP |
| NNT | LIVFYRHDCSTNGAP |
| VVK | KRHDESTQNGAP |
| NHT | LIVFYHDSTNAP |
| KNK | LVFYWDESCGA |
| BHK | LVFYHDESQAP |
| DNT | IVFYDSCTNGA |
| VVT | RHDSTNGAP |
| HHT | LIFYHSTNP |
| NWT | LIVFYHDN |
| RRK | KRDENSG |
| VRT | RHDSNG |
| HMT | YHSTNP |
| MRK | KRHNQS |
| TDK | LFYWC |
| BWT | LIFYHD |
| TKK | LFWC |
| RAK | KDEN |
| RRG | KREG |
| TWC | FY |
| RAG | KE |
| | |

| **G) Examples of triplets devoid of tyrosine-encoding codons** | |
|---|---|
| VNK | LIMVKRHDESTQNGAP |
| NNG | LMVWKRESTQGAP |
| VVK | KRHDESTQNGAP |
| MNK | LIMKRHSTQNP |
| VVT | RHDSTNGAP |
| RRK | KRDENSG |
| MRK | KRHNQS |
| VRT | RHDSNG |
| TKK | LFWC |
| RAK | KDEN |
| RRG | KREG |
| AVT | TSN |
| RAG | KE |
| | |

| **H) Examples of triplets devoid of tryptophan-encoding codons** | |
|---|---|
| VNK | LIMVKRHDESTQNGAP |
| NHK | LIMVFYKHDESTQNAP |
| NNT | LIVFYRHDCSTNGAP |
| VVK | KRHDESTQNGAP |
| BHK | LVFYHDESQAP |
| MNK | LIMKRHSTQNP |
| VVT | RHDSTNGAP |
| NWT | LIVFYHDN |
| RRK | KRDENSG |
| VRT | RHDSNG |
| MRK | KRHNQS |
| BWT | LIFYHD |
| RAK | KDEN |
| RRG | KREG |
| AVT | TSN |
| TWC | FY |
| RAG | KE |
| | |

| **I) Examples of triplets devoid of histidine-encoding codons** | |
|---|---|
| NNG | LMVWKRESTQGAP |
| NBK | LIMVFWRCSTGAP |
| NBK | LIMVFWRSTGAP |
| RRK | KRDENSG |
| TDK | LFYWC |
| TKK | LFWC |
| RAK | KDEN |
| RRG | KREG |
| KMT | YDSA |
| AVT | TSN |
| TWC | FY |
| RAG | KE |
| | |

| **J) Examples of triplets devoid of lysine and arginine-encoding codons** | |
|---|---|
| NHT | LIVFYHDSTNAP |
| KNK | LVFYWDESCGA |
| BHK | LVFYHDESQAP |
| DNT | IVFYDSCTNGA |
| HHT | LIFYHSTNP |
| NWT | LIVFYHDN |
| HMT | YHSTNP |
| TDK | LFYWC |
| BWT | LIFYHD |
| TKK | LFWC |
| KMT | YDSA |
| TWC | FY |

**Table 2:**

| Amino acids at randomized positions of clones isolated from EGF libraries 1 and 2. Arginine residues were not randomized in library EGF1 (indicated by -). | | | | | | |
|---|---|---|---|---|---|---|
| **library** | **clone** | **K28** | **R41** | **R45** | **K48** | **R43** |
| EGF1 | 3 | E | - | - | R | - |
| | 5 | Q | - | - | S | - |
| | 6 | A | - | - | A | - |
| | 8 | A | - | - | L | - |
| | 7 | V | - | - | L | - |
| | 2 | L | - | - | L | - |
| | 9 | V | - | - | G | - |
| | 12 | F | - | - | F | - |
| EGF2 | 2 | A | R | D | P | H |
| | 9 | S | R | R | A | P |
| | 4 | S | R | S | A | D |
| | 6 | Q | R | S | S | S |
| | 8 | Q | R | S | N | A |
| | 10 | Q | R | A | A | G |
| | 12 | Q | R | N | P | S |

## Claims

1. A polynucleotide selected from the group consisting of:
(a) polynucleotides encoding at least the mature modified epidermal growth factor (EGF) having the deduced amino acid sequence as shown in one of SEQ ID NOs 1-15;
(b) polynucleotides having the coding sequence, as shown in one of SEQ ID NOs: 16-30 encoding at least the mature modified EGF;
(c) polynucleotides encoding a fragment or derivative of a mature modified EGF encoded by a polynucleotide of any one of (a) to (b), wherein in said derivative one or more amino acid residues are conservatively substituted compared to said mature modified EGF with the proviso that polypeptide positions 28 and 48 are not Lys, and said fragment or derivative has epidermal growth factor receptor (EGFR) binding activity;
(d) polynucleotides which are at least 50% identical to a polynucleotide as defined in any one of (a) to (c) and which code for a modified EGF having EGFR binding activity; and
(e) polynucleotides the complementary strand of which hybridizes, preferably under stringent conditions to a polynucleotide as defined in any one of (a) to (d) and which code for a modified EGF having EGFR binding activity;
or the complementary strand of such a polynucleotide.

2. The polynucleotide of claim 1, wherein the modified EGF has at least wild-type EGFR binding activity.

3. The polynucleotide of claims 1 or 2, which is DNA, genomic DNA or RNA.

4. A vector containing the polynucleotide of one of claims 1 to 3.

5. The vector of claim 4 in which the polynucleotide is operatively linked to expression control sequences allowing expression in prokaryotic and/or eukaryotic host cells.

6. A host cell genetically engineered with the polynucleotide of one of claims 1 to 3 or the vector of claim 4 or 5.

7. A process for producing a modified EGF encoded by the polynucleotide of one of claims 1 to 3 comprising: culturing the host cell of claim 6 and recovering the modified EGF encoded by said polynucleotide.

8. A process for producing cells capable of expressing modified EGF comprising genetically engineering cells *in vitro* with the vector of claim 4 or 5, wherein said modified EGF is encoded by the polynucleotide of one of claims 1 to 3.

9. A modified EGF having the amino acid sequence encoded by the polynucleotide of one of claims 1 to 3 or obtainable by the process of claim 8.

10. The modified EGF of claim 9 or fusionpolypeptide thereof coupled to at least one chemical moiety, in particular through its N-terminal amino group.

11. The modified EGF or fusionpolypeptide of claim 10, wherein the chemical moiety is selected from the group consisting of a spacer, a marker, a tag, a lipid, in particular a phospholipid, a drug, a capping group, a polypeptide and a spacer attached to a second chemical moiety.

12. The modified EGF or fusionpolypeptide of claim 11, wherein the polypeptide is selected from the group consisting of a cytokine, a chemokine, a growth factor, an adhesion molecule, an antibody light and/or heavy chain, a single chain antibody, a toxin, an enzyme, a receptor ligand, a lytic peptide, a membrane insertion sequence and a fluorescent protein or fragments thereof.

13. The modified EGF or fusionpolypeptide of claim 11, wherein the spacer is selected from the group consisting of bifunctional polyethylenglycol and derivatives thereof, oligopeptides comprising between 1 to 40 natural or synthetic amino acids, 8-amino-3,6-dioxatanoic acid (doo), and (doo)ₙ, with n = 2-10.

14. The modified EGF or fusionpolypeptide of claim 11, wherein the marker is selected from the group consisting of an electron dense molecule, a paramagnetic molecule, a superparamagnetic molecule, a radioactive molecule, a non-radioactive isotope, and a fluorescent molecule.

15. The modified EGF or fusionpolypeptide of claim 11, wherein the lipid is selected from the group consisting of glycerides, glycerophospholipides, glycerophosphinolipids, glycerophosphono-lipids, sulfolipids, sphingolipids, phospholipids, isoprenolides, steroids, stearines, steroles, and carbohydrate containing lipids.

16. The modified EGF or fusionpolypeptide of claim 15, wherein the phospholipid is selected from the group consisting of phosphatidylcholine (PC), phosphatidylserine (PS), and phosphatidylethanolamine (PE), in particular distearoylphosphatidyl (DSPE) or alpha-(dipalmitoylphosphatidyl (DPP).

17. The modified EGF or fusionpolypeptide of claim 11, wherein the lipid is selected from the group consisting of N-caproylamine-PE, N-dodecanylamine-PE, phophatidylthioethanol, N-[4-(p-maleimidomethyl)cyclohexane-carboxamide-PE (N-MCC-PE), N-[4-(p-maleimidophenyl)butyramide]-PE (N-MPB), N-[3-(2-pyridyldithio)propionate]-PE (N-PDP), N-succinyl-PE, N-glutaryl-PE, N-dodecanyl-PE, N-biotinyl-PE, N-biotinyl-cap-PE, phosphatidyl-(ehtylene glycol), PE-polyethylene glycol (PEG)-carboxylic acid, PE-PEG-maleimide, PE-PEG-PDP, PE-PEG-amine, PE-PEG-biotin, PE-PEG-HNS, dipalmitoyl-glycerosuccinyl-lysine, alpha-methoxy-omega-(1,2-dioctadecenoyloxy glyceryl) (DO), alpa-methoxy-omega-(1,2-ditetradecenoyloxy glyceryl) (DT).

18. The modified EGF or fusionpolypeptide of claim 11, wherein the second chemical moiety is selected from the group consisting of a drug, a marker, a tag, a polypeptide and a lipid.

19. A composition comprising at least one modified EGF or fusionpolypeptide of one of claims 9 to 18 and at least one further component selected from the group consisting of liposomes, virosomes, microspheres, niosomes, dentrimers, stabilizers, buffers, excipients and additives.

20. The composition of claim 19, wherein the polypeptide is integrated into or attached to the liposome, microspheres, niosomes, dentrimers, or virosome.

21. The composition of claims 19 or 20 further comprising a drug selected from the group consisting of analgetics, antirheumatics, anthelminthics, antiallergics, antianemics, antiarrhythmics, antibiotics, antiinfectives, antidemenics (nootropics), antidiabetics, antidotes, antiemetics, antivertiginosics,, antiepileptics, antihemorrhagics, antihypertonics, antihypotonics, anticoagulants, antimycotics, antitussiv agents, antiviral agents, beta-receptor and calcium channel antagonists, broncholytic and antiastmatic agents, chemokines, cytokines, mitogens, cytostatics, cytotoxic agents and prodrugs thereof, dermatics, hypnotics and sedatives, immunosuppressants, immunostimulants, peptide or protein drugs, in particular hormones and physiological or pharmacological inhibitors of mitogens, chemokines, or cytokines or their respective prodrugs. Of course it is also envisioned that a liposome of the invention comprises more than one drug at once.

22. The compositions of claim 21, wherein the cytostatics and cytotoxic drugs are selected from the group consisting of alkylating substances, anti-metabolites, antibiotics, epothilones, anti-androgens, anti-estrogens, platinum compounds, hormones and antihormones, interferons and inhibitors of cell cycle-dependent protein kinases (CDKs), platine coordination complexes, ethyleneimenes, methylmelamines, trazines, vinca alkaloids, pyrimidine analogs, purine analoga, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, tamoxifen, taxol, teniposide, tertiposide, testolactone, testosteronpropionate, thioguanine, thiotepa, tinidazole, topotecan, triaziquone, treosulfan, trimethoprim, trofosfamide, vinblastine, vincristine, vindesine, vinblastine, vinorelbine, and zorubicin, or their respective derivatives or analogs thereof.

23. Use of a modified EGF or of a fusionpolypeptide of one of claims 9 to 18 or of a composition of one of claims 19 to 22 for the production of a medicament for the therapy of a diseases selected from the group consisting of proliferative diseases, immune diseases, infectious diseases, vascular diseases, rheumatoid diseases, and diseases, in which cells in or adjacent to the disease site show an increased expression of EGFR.

24. The use of claim 23, wherein the proliferative disease is selected from the group consisting of lung cancer, liver cancer, head and neck cancer, bladder, cancer, prostate cancer, cervix cancer, endometrial cancer, colorectal adenoma and adenocarcinoma, gastric cancer, oesophageal cancer, breast cancer, squamous carcinoma, glioblastomas and other high-grade primary brain tumors, chronic inflammatory proliferative diseases, vascular proliferative diseases and virus-induced proliferative diseases.

25. The use of modified EGF or fusionpolypeptide of one of claims 9 to 18 or of a composition of one of claims 19 to 23 for the diagnosis of diseases selected from the group consisting of proliferative diseases, immune diseases, infectious diseases, vascular diseases, rheumatoid diseases, and diseases, in which cells in or adjacent to the disease site show an increased expression of EGFR.

26. A method for producing a modified binding polypeptide, which is suitable for site-directed coupling, comprising the step of:
modifying a polynucleotide encoding the binding polypeptide, which is to be modified, by identifying within the reading frame of the polynucleotide all codons with the sequence:
a) AAA and AAG encoding Lys and replacing this (these) codon(s) with (a) codon(s) NNN excluding AAA and AAG;
b) AAA and AAG encoding Lys and replacing this (these) codon(s) with (a) codon(s) NNN excluding AAA and AAG and all codons with the sequence CGT, CGC, CGA, CGG, AGA, and AGG encoding Arg and replacing this (these) codon(s) with (a) codon(s) NNN excluding CGT, CGC, CGA, CGG, AGA, and AGG;
c) GAT and GAC encoding Asp and replacing this (these) codon(s) with (a) codon(s) NNN excluding GAT and GAC and all codons with the sequence GAA and GAG encoding Glu and replacing this (these) codon(s) with (a) codon(s) NNN excluding GAA and GAG;
d) TGT and TGC encoding Cys and replacing all but one of this (these) codon(s) with (a) codon(s) NNN excluding TGT and TGC;
e) TCT, TCC, TCA, TCG, AGT and AGC encoding Ser and replacing all but one of this (these) codon(s) with (a) codon(s) NNN excluding TCT, TCC, TCA, TCG, AGT and AGC and all codons with the sequence ACT, ACC, ACA and ACG encoding Thr and replacing all but one of this (these) codon(s) with (a) codon(s) NNN excluding ACT, ACC, ACA and ACG;
f) ATG encoding Met and replacing all but one of this (these) codon(s) with (a) codon(s) NNN excluding ATG;
g) TAT and TAC encoding Tyr and replacing all but one of this (these) codon(s) with (a) codon(s) NNN excluding TAT and TAC;
h) TGG encoding Trp and replacing all but one of this (these) codon(s) with (a) codon(s) NNN excluding TGG; and/or
i) CAT and CAC encoding His and replacing all but one of this (these) codon(s) with (a) codon(s) NNN excluding CAT and CAC;
wherein N has the meaning: A, C, G or T.

27. The method of claim 26, wherein all codons with the sequence
j) AAA and AAG encoding Lys are replaced with a sequence selected from the group consisting of BNK, NNT, NBK, NBK, KNK, NHT, BHK, DNT, VVT, HHT, VRT, HMT, TDK, BWT, TKK, TWC, KMT, AVT, and TWC;
k) AAA and AAG encoding Lys and CGT, CGC, CGA, CGG, AGA, and AGG encoding Arg are replaced with a sequence selected from the group consisting of NHT, KNK, BHK, DNT, HHT, NWT, HMT, TDK, BWT, TKK, KMT, and TWC;
l) AAA and AAG encoding Lys are replaced with a sequence selected from the group consisting of BNK, NNT, NBK, NBK, KNK, NHT, BHK, DNT, VVT, HHT, VRT, HMT, TDK, BWT, TKK, TWC, KMT, AVT, and TWC and all codons with the sequence CGT, CGC, CGA, CGG, AGA, and AGG encoding Arg are replaced with a sequence selected from the group consisting of NHK, NHT, KNK, BHK, DNT, HHT, NWT, HMT, BWT, TDK, TKK, RAK, and TWC;
m) GAT and GAC encoding Asp and GAA and GAG encoding Glu are replaced with a sequence selected from the group consisting of HNK, NBK, MNK, HHT, MRK, TKK, and TWC, and;
n) TGT and TGC encoding Cys are replaced all but one of with a sequence selected from the group consisting of VNK, NHK, NNG, VVK, BHK, MNK, VVT, NWT, RRK, VRT, MRK, BWT, NKT, RAK, RRG, KMT, AVT, and RAG,;
o) TCT, TCC, TCA, TCG, AGT and AGC encoding Ser and ACT, ACC, ACA and ACG encoding Thr are replaced all but one of with a sequence selected from the group consisting of RAK, TDK, TKK, VWT, BWT, NWT, RRG, and TWC;
p) ATG encoding Met are replaced all but one of with a sequence selected from the group consisting of NVK, BNK, NNT, VVK, NHT, KNK, BHK, DNT, VVT, HHT, NWT, RRK, VRT, HMT, MRK, TDK, BWT, TKK, RAK, RRG, TWC, and RAG;
q) TAT and TAC encoding Tyr are replaced all but one of with a sequence selected from the group consisting of VNK, NNG, VVK, MNK, VVT, RRK, MRK, VRT, TKK, RAK, RRG, AVT, and RAG;
r) TGG encoding Trp are replaced all but one of with a sequence selected from the group consisting of VNK, NHK, NNT, VVK, BHK, MNK, VVT, NWT, RRK, VRT, MRK, BWT, RAK, RRG, AVT, TWC, and RAG; and/or
s) CAT and CAC encoding His are replaced all but one of with a sequence selected from the group consisting of NNG, NBK, NBK, RRK, TDK, TKK, RAK, RRG, KMT, AVT, TWC, and RAG;
wherein R has the meaning: A or G; K has the meaning: G or T; M has the meaning: A or C; W has the meaning: A or T; B has the meaning: C, G or T; D has the meaning: A, G or T; H has the meaning: A, C or T; V has the meaning: A, G or C; N has the meaning: A, C, G or G.

28. The method of claims 26 or 27, further comprising the step of coupling the modified polynucleotide to at least one additional polynucleotide encoding a polypeptide to produce a polynucleotide encoding a modified fusion polypeptide.

29. The method of one of claims 26 to 28, further comprising the step of expressing the modified polynucleotide to produce a modified binding polypeptide or modified binding fusion polypeptide.

30. The method of claim 29, further comprising the steps of:
a) incubating the modified binding polypeptide or fusion polypeptide or viral particles or cells displaying the modified binding polypeptide or fusion polypeptide with at least one binding partner of the binding polypeptide, and
b) selecting the modified binding polypeptide or fusion polypeptides or viral particles or cells displaying the modified binding polypeptide or fusion polypeptide, which shows at least 10% of the binding strength of the binding polypeptide to the binding partner.

31. The method of one of claims 26 to 30, further comprising the step of site specific coupling of the modified binding polypeptide or fusion polypeptide to at least one chemical moiety.

32. The method of claim 31, wherein the chemical moiety is coupled to
aa) a N-terminal amino group of the modified binding polypeptide or fusion polypeptide and wherein the modified binding polypeptide has been modified according to alternative a) or b) of claim 26 or according to alternative j) to l) of claim 27;
bb) a C-terminal carboxyl group of the modified binding polypeptide or fusion polypeptide and wherein the modified binding polypeptide has been modified according to alternative c) of claim 26 or according to alternative m) of claim 27;
cc) a newly added Cys residue of the modified binding polypeptide or fusion polypeptide and wherein the modified binding polypeptide has been modified according to alternative d) of claim 26 or according to alternative n) of claim 27;
dd) a newly added Ser residue of the modified binding polypeptide or fusion polypeptide and wherein the modified binding polypeptide has been modified according to alternative e) of claim 26 or according to alternative o) of claim 27;
ee) a newly added Met residue of the modified binding polypeptide or fusion polypeptide and wherein the modified binding polypeptide has been modified according to alternative f) of claim 26 or according to alternative p) of claim 27;
ff) a newly added Tyr residue of the modified binding polypeptide or fusion polypeptide and wherein the modified binding polypeptide has been modified according to alternative g) of claim 26 or according to alternative q) of claim 27;
gg) a newly added Trp residue of the modified binding polypeptide or fusion polypeptide and wherein the modified binding polypeptide has been modified according to alternative h) of claim 26 or according to alternative r) of claim 27; and/or
hh) a newly added His residue of the modified binding polypeptide or fusion polypeptide and wherein the modified binding polypeptide has been modified according to alternative i) of claim 26 or according to alternative s) of claim 27.

33. The method of claims 31 or 32, wherein the chemical moiety is selected from the group consisting of a spacer, a marker, a tag, a lipid, in particular a phospholipid, a drug, a capping group, a polypeptide and a spacer attached to a second chemical moiety.

34. The method of claim 33, wherein polypeptide to is selected from the group consisting of a cytokine, a chemokine, a growth factor, an adhesion molecule, an antibody light and/or heavy chain, a single chain antibody, a toxin, an enzyme, a receptor ligand, a lytic peptide, a membrane insertion sequence and a fluorescent protein or fragments thereof.

35. The method of one of claims 26 to 34, wherein the binding polypeptide is selected from the group consisting of growth factors, in particular VEGF, EGF, Her2/neu, PDGF, TGFα, TGFβ, KGF, SDGF, FGF, IGF, HGF, NGF, BDNF, neurotrophine, BMF, bombesin, M-CSF, GM-CSF, thrombopoietin, erythropoietin, SCF, SDGF, oncostatin, PDEGF, endothelin; cytokines, in particular IL-1, IL-2, IL-3, IL-4, IL-5, IL-6, IL-7, IL-8, IL-9, IL-10, IL-12, IL-13, IL-14, IL-15, interferon α, β or γ, tumor necrosis factors such as TNFα, TNFβ; chemokines, in particular RANTES, MCAF, MIP-1α or β, NAP, β-thromboglobulin; peptide hormones such as SRH, SIH, STH, MRH, MSH, PRH, PIH, prolactin, LH-RH, FSH-RH, LH/ICSH, FSH, TRH, TSH, CRH, ACTH, agiotensin, kinine, histamine; adhesion molecules, in particular LFA-1, MAC-1, VLA-4, PECAM, vitronectin, GMP-140, ICAM-1, VCAM-1, fibronectin, laminin, B7, CD28, CD40, CD40L and selectins; viral coat proteins; and bacterial surface proteins.

36. Use of a modified binding polypeptide or fusion polypeptide producable by a method according to one of claims 26 to 35 for the manufacture of a medicament or diagnostic for the prevention, treatment or diagnosis of a disease, which is **characterized by** an increased or decreased amount of at least one binding partner of the binding polypeptide in diseased tissue or cells involved in the disease.

37. Use according to claim 36, wherein the disease is selected from the group of diseases consisting of proliferative diseases, immune diseases, infectious diseases, vascular diseases and rheumatoid diseases.
